# EUROPEAN PATENT APPLICATION

(11) **EP 4 717 765 A1**
(43) Date of publication of application: **01.04.2026**
(21) Application number: 24811087.6
(22) Date of filing: 21.05.2024
(51) Int. Cl.: C12N 5/0783, A61K 35/17, A61K 35/545, A61P 35/00, A61P 37/04, C12N 5/10

(54) **METHOD FOR PRODUCING CELL POPULATION CONTAINING CD4 SINGLE POSITIVE HELPER T CELLS FROM PLURIPOTENT STEM CELLS**

(30) Priority: 22.05.2023 JP 2023084219
(71) Applicant: Kyoto University, Kyoto-shi, Kyoto 606-8501 (JP); Shinobi Therapeutics Co. Ltd., Kyoto-shi, Kyoto, 606-8304 (JP)
(72) Inventor: KANEKO, Shin, Kyoto-shi, Kyoto 606-8501 (JP); KAWAI, Yohei, Kyoto-shi, Kyoto 606-8501 (JP); ZHANG, Chaoqi, Kyoto-shi, Kyoto 606-8501 (JP); HITOSHI, Yasumichi, Kyoto-shi, Kyoto 606-8304 (JP)
(74) Representative: Zellentin & Partner mbB Patentanwälte
(86) International application number: PCT/JP2024/018570
(87) International publication number: WO 2024/242080

(57) **Abstract**

[Problem] To provide a method for efficiently producing a cell population containing functionally superior CD4 single-positive helper T cells from pluripotent stem cells.

[Solution] A method for producing a cell population containing CD4 single-positive, CD8-negative helper T cells, comprising: (1) culturing hematopoietic stem cells induced to differentiate from pluripotent stem cells in the presence of a T cell differentiation inducing factor; (2) culturing the cells obtained in step (1) in the presence of a T cell differentiation inducing factor and a cell adhesion factor; and (3) culturing the cells obtained in step (2) in the presence of the cell adhesion factor but in the absence of the T cell differentiation inducing factor.

## Description

### TECHNICAL FIELD

The present invention relates to a method for producing a cell population containing CD4 single positive helper T cells from pluripotent stem cells, particularly induced pluripotent stem cells (iPS cells), a pharmaceutical agent containing the cell population, an agent for killing cells expressing tumor-associated antigens and an agent for preventing or treating cancer in a mammal, each containing the cell population, and a method for preventing or treating cancer in a mammal, which comprises administering an effective amount of the cell population.

### BACKGROUND

In recent years, active research has been conducted into cancer immunotherapy, a new cancer treatment method that eliminates cancer by activating the patient's immune system. In this cancer immunotherapy, T cells that specifically recognize and attack cancer are crucial. However, the number of T cells that recognize cancer is low within a patient's body. Therefore, research is underway into cancer immunotherapy that uses genetically modified T cells that express receptors on their cell surface that can specifically recognize cancer antigens or tumor-associated antigens. Such genetically modified T cells have been reported to express chimeric antigen receptors (CARs) and T cell receptors (TCRs) that target specific cancer antigens or tumor-associated antigens; these are referred to as CAR-T cells and TCR-T cells, respectively (Patent Documents 1 and 2).

CAR is a fusion protein consisting of the antigen-recognition portion of an antibody that specifically recognizes a cancer antigen or tumor-associated antigen and an intracellular domain derived from a TCR. CAR-T cells are able to recognize antigens expressed on the cell surface in a manner that is not restricted by human leukocyte antigens (HLA). For this reason, CAR-T cells are expected to be used in allogeneic transplants as well as autologous transplants. TCR is the receptor used by T cells to recognize antigens and is composed of a dimer of α and β chains, or γ and δ chains. TCR forms a complex with CD3 molecules on the surface of T cells and activates T cells by recognizing antigen molecules bound to major histocompatibility complex (MHC) molecules.

T cells are broadly classified into killer T cells, which are CD8 positive T cells that directly recognize tumors and induce apoptosis, and helper T cells, which are CD4 positive T cells that enhance the function of killer T cells and other lymphocytes such as B cells. Cancer immunotherapy has focused on CD8 positive T cells, which can directly recognize tumor cells. This is because, while tumor cells that endogenously express MHC class II molecules become a direct target for CD4 positive T cells, most tumors do not express MHC class II molecules and are therefore not directly recognized by CD4 positive T cells. However, tumor antigens can be captured and cross-presented by tumor stromal cells expressing MHC class II molecules (Non-Patent Document 1). Necrotic tumor cells or vesicles released from tumor cells are taken up by stromal cells and primarily enter the classical processing pathway for MHC class II molecules, so cross-presentation of tumor antigens on MHC class II molecules is speculated to be more effective than that on MHC class I molecules (Non-Patent Document 2). In most solid tumors, monocytes/macrophages are the most abundant MHC class II molecule-positive cells. Depending on the presence or absence of MHC class II molecules on tumor cells and the CD4-positive T cell subset, several different tumor rejection mechanisms have been proposed (Non-Patent Document 1).

In cancer immunotherapy using genetically modified T cells, difficulties in securing sufficient numbers of T cells, as well as T cell exhaustion, such as a reduced proliferation capacity and a reduced immune response to antigens on tumor cells, pose obstacles to the effective implementation of cancer immunotherapy. To overcome these obstacles and facilitate the implementation of cancer immunotherapy, techniques have been reported in which induced pluripotent stem cells (iPS cells), established from antigen-specific T cells isolated from peripheral blood mononuclear cells, are expanded and then differentiated into CD8-positive T cells (Patent Documents 3 and 4, and Non-Patent Documents 3 and 4). Because these techniques enable the continuous induction of large numbers of functional T cells from iPS cells, they are thought to be an important foundation for T-cell-based cancer immunotherapy.

In cancer immunotherapy using genetically modified T cells expressing tumor-reactive TCRs or CARs, it has been reported that combining the most potent CD4 positive and CD8 positive CAR-expressing T cell subsets can achieve synergistic in vivo antitumor effects (Non-Patent Document 5). Therefore, when inducing functional T cells from iPS cells, it is important to produce not only CD8-positive T cells but also CD4-positive T cells. However, the reported techniques described above produce only CD8-positive T cells from iPS cells, and are unable to produce CD4-positive T cells or long-lived naive T cells. A method for producing CD4-positive T cells from iPS cells has been reported in which the CD4 gene is introduced into iPS cell-induced T cells and the cells are cultured in a culture medium containing interleukin-2 and interleukin-15 (Patent Document 5). Another method for producing CD4-positive T cells has also been reported, in which iPS cells are cultured in an artificial thymic organ (ATO) (Non-Patent Document 6), but the efficiency of CD4-positive T cell production is low.

### PRIOR ART

### Patent Documents

[Patent Document 1] International Publication WO2013/070468
[Patent Document 2] International Publication WO2015/173112
[Patent Document 3] International Publication WO2011/096482
[Patent Document 4] International Publication WO2018/135646
[Patent Document 5] International Publication WO2018/168829

### Non-Patent Documents

[Non-Patent Document 1] Poncette L, et al. The role of CD4 T cells in rejection of solid tumors. Curr. Opin. Immunol. 2022; 74:18-24.
[Non-Patent Document 2] Blum JS, et al. Pathways of antigen processing. Ann. Rev. Immunol. 2013; 31:443-473.
[Non-Patent Document 3] Nishimura T, et al. Production of rejuvenated antigen-specific T cells by reprogramming to pluripotency and redifferentiation. Cell Stem Cell. 2013; 12:114-126.
[Non-Patent Document 4] Minagawa A, et al. Enhancing T cell receptor stability in rejuvenated iPSC-derived T cells improves their use in cancer immunotherapy. Cell Stem Cell. 2018; 23:850-858.
[Non-Patent Document 5] Sommermeyer D, et al. Chimeric antigen receptor-modified T cells derived from defined CD8+ and CD4+ subsets confer superior antitumor reactivity in vivo. Leukemia. 2016; 30:492-500.
[Non-Patent Document 6] Montel-Hagen A, et al. Organoid-induced differentiation of conventional T cells from human pluripotent stem cells. Cell Stem Cell. 2019; 24:376-389.

### Summary of the Invention

### Problem to be Solved by the Invention

Current cancer immunotherapy using autologous transplantation of CAR-T cells has demonstrated excellent clinical efficacy. This is because CAR is introduced not only into killer T cells in the peripheral blood but also into helper T cells, which then participate in the immune response. However, in order to perform stable and effective cancer immunotherapy without relying on autologous cells, the challenge is to stably produce helper T cells from pluripotent stem cells.

The present invention aims to provide a method for efficiently producing a cell population containing functionally superior CD4 single-positive helper T cells from pluripotent stem cells. Furthermore, the present invention aims to provide a pharmaceutical containing the cell population produced by the method, an agent for killing cells expressing tumor-associated antigens, and an agent for preventing or treating cancer in mammals, as well as a method for preventing or treating cancer in mammals, which comprises administering an effective amount of the cell population produced by the method.

### Means for Solving the Problems

The inventors discovered that CD4 single-positive helper T cells can be efficiently produced by selecting whether or not to use a T cell differentiation-inducing factor and a cell adhesion factor for each step in the process of inducing differentiation of pluripotent stem cells, and thus completed the present invention.

The inventors further discovered that CD4 single-positive helper T cells can be produced by culturing cells using a Wnt pathway activator and selecting whether or not to use a T cell differentiation-inducing factor, a cell adhesion factor, and an anti-CD3 antibody for each step in the process of inducing differentiation of pluripotent stem cells, and thus completed the present invention.

That is, the present invention provides the following:
[1] A method for producing a cell population containing CD4-single positive, CD8-negative helper T cells, comprising:
   (1) a step of culturing hematopoietic stem cells, differentiated from pluripotent stem cells, in the presence of a T cell differentiation-inducing factor;
   (2) a step of culturing the cells obtained in step (1) in the presence of a T cell differentiation-inducing factor and a cell adhesion factor; and
   (3) a step of culturing the cells obtained in step (2) in the presence of the cell adhesion factor but in the absence of the T cell differentiation-inducing factor.
[2] The method according to [1], wherein the cell culture in steps (1), (2), and (3) is carried out in the presence of a Wnt/β-catenin pathway activator.
[3] The method according to [1] or [2], further comprising the step of confirming that the cells obtained in step (1) contain CD3-, CD1a-, and CD5-positive precursor T cells.
[4] The method according to [1] or [2], further comprising the step of confirming that the cells obtained in step (2) contain CD3-positive and CD4/CD8 double-positive T cells.
[5] The method according to [4], wherein the CD3-positive and CD4/CD8-double-positive T cells are further CD5- and CD1a-positive.
[6] The method according to [1] or [2], wherein the cells obtained in step (3) are mature helper T cells that are CD1a- and CD8-negative and CD5-positive and CD4-single-positive, or a mixture of the mature helper T cells and mature killer T cells that are CD5- and CD8-positive and CD1a- and CD4-negative.
[7] The method according to [1] or [2], further comprising the step of confirming that the cells obtained in step (3) include T cells that highly express CD28.
[8] The method according to [7], wherein the CD28 expression level of the T cells highly expressing CD28 is equivalent to the CD28 expression level of T cells present in human peripheral blood.
[9] A method for producing a cell population containing CD4 single-positive, CD8-negative helper T cells, comprising:
   (1) culturing hematopoietic stem cells, differentiated from pluripotent stem cells, in the presence of a T cell differentiation inducing factor and a Wnt/β-catenin pathway activator;
   (2) culturing the cells obtained in step (1) in the presence of a cell adhesion factor, a Wnt pathway activator, and an anti-CD3 antibody, but in the absence of a T cell differentiation inducing factor; and
   (3) culturing the cells obtained in step (2) in the presence of a cell adhesion factor and a Wnt pathway activator, but in the absence of a T cell differentiation inducing factor and an anti-CD3 antibody.
[10] The method according to [9], wherein the cells obtained in step (3) are a mixture of mature helper T cells that are CD1a and CD8 negative, and CD5 positive and CD4 single positive, and mature killer T cells that are CD5 and CD8 positive, and CD1a and CD4 negative.
[11] The method according to [1] or [9], wherein the T cell differentiation inducing factor is a Notch ligand.
[12] The method according to [11], wherein the Notch ligand is DLL4 or DLL4-Fc.
[13] The method according to [1] or [9], wherein the cell adhesion factor is fibronectin or a fragment thereof, or RetroNectin (registered trademark).
[14] The method according to [2] or [9], wherein the Wnt pathway activator is a GSK-3 inhibitor.
[15] The method according to [14], wherein the GSK-3 inhibitor is CHIR-99021.
[16] The method according to [1] or [9], wherein the T cell differentiation factor and the cell adhesion factor are present by coating a cell culture substrate.
[17] The method according to [16], wherein the cell culture substrate is cell culture beads or a cell culture plate.
[18] The method according to [1] or [2], wherein the cell culture in steps (1) and (2) is performed in the presence of IL-7 and FLT3L.
[19] The method according to [1] or [2], wherein the cell culture in steps (1) and (2) is performed in the presence of IL-7, FLT3L, 2-phospho-L-ascorbic acid, and SDF-1α and/or a p38 inhibitor.
[20] The method according to [1] or [2], wherein the cell culture in steps (1) and (2) is performed in the presence of IL-7, FLT3L, SDF-1α, and a p38 inhibitor.
[21] The method according to [1] or [2], wherein the cell culture in steps (1) and (2) is performed in the presence of IL-7, FLT3L, 2-phospho-L-ascorbic acid, SDF-1α, a p38 inhibitor, and SCF.
[22] The method according to [1] or [2], wherein the cell culture in step (3) is performed in the presence of IL-7.
[23] The method according to [1] or [2], wherein the cell culture in step (3) is carried out in the presence of IL-7, 2-phospho-L-ascorbic acid, and SDF-1α and/or a p38 inhibitor.
[24] The method according to [1] or [2], wherein the cell culture in step (3) is carried out in the presence of IL-7, SDF-1α, and a p38 inhibitor.
[25] The method according to [1] or [2], wherein the cell culture in step (3) is carried out in the presence of IL-7, 2-phospho-L-ascorbic acid, SDF-1α, a p38 inhibitor, and SCF.
[26] The method according to [1] or [2], wherein the cell culture in steps (1) and (2) is performed while maintaining the cell density at subconfluence.
[27] The method according to [9], wherein the cell culture in step (1) is performed in the presence of IL-7, FLT3L, 2-phospho-L-ascorbic acid, SDF-1α, a p38 inhibitor, SCF, and TPO.
[28] The method according to [9], wherein the cell culture in steps (2) and (3) is performed in the presence of IL-7, 2-phospho-L-ascorbic acid, SDF-1α, and a p38 inhibitor.
[29] The method according to [1] or [9], wherein the pluripotent stem cells are human iPS cells.
[30] The method according to [29], wherein the human iPS cells are human peripheral blood mononuclear cells from which B cells and T cells have been depleted, or iPS cells obtained by reprogramming T cells.
[31] A pharmaceutical comprising a cell population produced by the method according to [1] or [9].
[32] The pharmaceutical according to [31], for use in the prevention or treatment of cancer.
[33] An agent for killing cells expressing a tumor-associated antigen, comprising a cell population produced by the method according to [1] or [9].
[34] A method for preventing or treating cancer in a mammal, comprising administering to the mammal an effective amount of a cell population produced by the method described in [1] or [9].
[35] A method for preventing or treating cancer in a mammal, comprising administering to the mammal an effective amount of the medicament described in [31].
[36] A method for preventing or treating cancer in a mammal, comprising administering to the mammal an effective amount of the killing agent described in [33].
[37] An agent for preventing or treating cancer in a mammal, comprising a cell population produced by the method described in [1] or [9].
[38] Cells produced by the method described in [1] or [9] for use in the prevention or treatment of cancer.
[39] Cells produced by the method described in [1] or [9] for the production of a cancer preventive or therapeutic agent.

In the specification of this application, the method described in [1] is referred to as the first method, and the method described in [9] is referred to as the second method.

### Effects of the Invention

CD4 single-positive helper T cells are necessary for enhancing tumor immunity, but based on previously reported technologies, T cells produced from pluripotent stem cells are primarily CD8 single-positive killer T cells, and CD4 single-positive helper T cells cannot be efficiently produced. According to the present invention, hematopoietic stem cells differentiated from pluripotent stem cells are cultured in the presence of a T cell differentiation inducing factor, then in the presence of a T cell differentiation inducing factor and a cell adhesion factor, and finally in the presence of a cell adhesion factor but in the absence of a T cell differentiation inducing factor. This allows for the production of a cell population containing CD4 single-positive helper T cells without the need for exogenous CD4 gene introduction. Furthermore, according to the present invention, a cell population containing CD4 single-positive helper T cells and CD8 positive killer T cells can be produced. Furthermore, by culturing the hematopoietic stem cells in the presence of a Wnt/β-catenin pathway activator throughout the entire culture period, the proportion of CD4 single-positive helper T cells in the cell population can be increased.

Furthermore, according to the present invention, hematopoietic stem cells differentiated from pluripotent stem cells can be cultured in the presence of a T cell differentiation inducing factor and a Wnt/β-catenin pathway activator, then in the presence of a cell adhesion factor, a Wnt pathway activator, and an anti-CD3 antibody, but in the absence of a T cell differentiation inducing factor, and finally in the presence of a cell adhesion factor and a Wnt pathway activator, but in the absence of a T cell differentiation inducing factor and an anti-CD3 antibody, thereby producing a cell population containing CD4 single-positive helper T cells and CD8 positive killer T cells.

CD4 single-positive helper T cells produced by the method of the present invention have superior cell proliferation properties compared to CD8 single-positive killer T cells, and also have superior cancer cell-killing and in vivo tumor-suppressing effects. Pharmaceuticals containing cell populations containing CD4 single-positive helper T cells produced by the method of the present invention, or agents for killing cells expressing tumor-associated antigens, are useful for the prevention or treatment of cancer.

### Brief Description of the Drawings

Figure 1 shows the results of a flow cytometric analysis of the effects of coating cell culture plates with DLL4-Fc and RetroNectin in each step on the production of CD4 single-positive helper T cells from iPS cells.
Figure 2 shows the results of a flow cytometric analysis of the effects of coating cell culture plates with DLL4-Fc and RetroNectin in step (3) on the production of CD4 single-positive helper T cells from iPS cells.
Figure 3 shows a comparison of the coating conditions in step (3) with the amount of CD4 single-positive helper T cells produced from iPS cells.
Figure 4 shows the results of flow cytometry analysis of the effects of coating cell culture plates with DLL4-Fc and retronectin in steps (1) and (2) on the production of CD4 single-positive helper T cells from iPS cells.
Figure 5 shows a comparison of the coating conditions in steps (1) and (2) and the amount of CD4 single-positive helper T cells produced from iPS cells.
Figure 6 shows the effect of a Wnt pathway activator (CHIR-99021) on the production of CD4 single-positive helper T cells from iPS cells.
Figure 7 shows the results of flow cytometry analysis of CD40L expression in response to TCR stimulation in CD4 single-positive helper T cells matured from iPS cells by DLL4-Fc/retroNectin switching treatment and anti-CD3 antibody treatment in the coating of cell culture plates.
Figure 8 shows the results of flow cytometry analysis of CD40L expression in response to TCR stimulation in CD4 single-positive helper T cells and CD8 single-positive killer T cells that have undergone different stages of maturation.
Figure 9 shows the expression of Thpok and PLZF in CD4 single-positive helper T cells and CD8 single-positive killer T cells produced from iPS cells by DLL4-Fc/retroNectin switching in the coating of cell culture plates, measured by TaqMan qPCR, and compared with the results measured in primary T cells of the adaptive immune system obtained from peripheral blood.
Figure 10 shows the results of measuring Thpok and Runx3 expression in CD4 single-positive helper T cells and CD8 single-positive killer T cells that have undergone different maturation processes, measured by TaqMan qPCR.
Figure 11 shows the results of measuring the proliferation of CD4 single-positive helper T cells and CD8 single-positive killer T cells that have undergone different maturation processes.
Figure 12 shows flow cytometric analysis of naive markers (CD28) and adaptive immune markers (CD2 and CD5) expressed by CD4 single-positive helper T cells and CD8 single-positive killer T cells produced from iPS cells by DLL4-Fc/retroNectin switching in the coating of cell culture plates after feeder-free expansion culture, and compares the results with those of the T cells before expansion culture and fresh human peripheral blood mononuclear cells.
Figure 13A shows the results of flow cytometric analysis of EGFR expression in the resulting cells after gene transfer of anti-CD19 CAR (anti-CD19 CAR-IRES-EGFR) using retroviral into a cell population containing CD4 single-positive helper T cells and CD8 single-positive killer T cells, which were produced from iPS cells by DLL4-Fc/retronectin switching in the coating of a cell culture plate.
Figure 13B shows the results of measuring the amounts of cytokines (IFN-y, IL-2, and TNFα) in the culture supernatant after 24 hours of co-culture of CD4 single-positive helper T cells and CD8 single-positive killer T cells, which were produced from iPS cells by DLL4-Fc/retroNectin switching in the coating of a cell culture plate and were transfected with anti-CD19 CAR-IRES-EGFR, with the CD19-positive human pre-B cell leukemia cell line Nalm6.
Figure 14 shows the killing activity of CD4 single-positive helper T cells and CD8 single-positive killer T cells produced from iPS cells by DLL4-Fc/retronectin switching in the coating of cell culture plates against the human pre-B cell leukemia cell line Nalm6.
Figure 15 shows the effects of IL-7 and IL-15 on the killing activity of CD4 single-positive helper T cells and CD8 single-positive killer T cells produced from iPS cells by DLL4-Fc/retronectin switching in the coating of cell culture plates against the human pre-B cell leukemia cell line Nalm6.
FIG. 16A shows the results of measuring the cytotoxic activity against the CD19-positive human pre-B cell leukemia cell line Nalm6 after introducing anti-CD19 CAR into CD4 single-positive helper T cells and CD8 single-positive killer T cells prepared from iPS cells by DLL4-Fc/retroNectin switching in the coating of a cell culture plate, T cells prepared from iPS cells prepared by artificial thymus organ (ATO) culture, and primary T cells derived from a healthy donor.
Figure 16B shows the results of measuring the cytotoxic activity against the CD19-negative human leukemia cell line K562 after introducing anti-CD19 CAR into CD4 single-positive helper T cells and CD8 single-positive killer T cells prepared from iPS cells by DLL4-Fc/retroNectin switching in the coating of a cell culture plate, T cells prepared from iPS cells prepared by artificial thymus organ (ATO) culture, and primary T cells derived from a healthy donor.
Figure 17A shows the effect of Nalm6 on the pharmacokinetics of the mice measured by in vivo imaging using luciferase luminescence after intravenously injection of CD4 single-positive helper T cells and CD8 single-positive killer T cells generated from iPS cells by DLL4-Fc/retronectin switching in the coating of cell culture plates into NSG mice transplanted with the human pre-B cell leukemia cell line Nalm6.
Figure 17B shows the Kaplan-Meier survival curves for the NSG mice described in Figure 17A.
Figure 18A shows the result of flow cytometric analysis of CD4 single-positive helper T cells produced from hematopoietic stem cells derived from human iPS cells in the presence of a Wnt pathway activator (CHIR-99021).
Figure 18B shows the result of flow cytometric analysis of CD5, TCR α/β chain, and CD1a expression in the CD4 single-positive T cell subset shown in Figure 18A.
Figure 19 shows the results of flow cytometry analysis of the effect of a Wnt pathway activator (CHIR-99021) on the production of CD4 single-positive helper T cells from iPS cell-derived hematopoietic stem cells.
Figure 20A shows the percentage of mature CD8 single-positive killer T cells and cell yield among T cells produced from iPS cell-derived hematopoietic stem cells in the presence of a Wnt pathway activator (CHIR-99021).
Figure 20B shows the percentage of mature CD4 single-positive helper T cells and cell yield among T cells produced from iPS cell-derived hematopoietic stem cells in the presence of a Wnt pathway activator (CHIR-99021).
Figure 21 shows the results of flow cytometric analysis of CD4 and CD8 expression in mature T cells produced from hematopoietic stem cells derived from iPS cells in the presence of a Wnt pathway activator (CHIR-99021), and the results of flow cytometric analysis of CD4 and CD8 expression in CD4 and CD8 single-positive T cells after flow cytometric sorting and subsequent expansion cultureing using anti-CD3 and anti-CD28 antibody-conjugated beads.
Figure 22 shows the results of flow cytometry analysis of CD40L expression in CD4 single-positive helper T cells produced from hematopoietic stem cells derived from iPS cells in the presence of a Wnt pathway activator (CHIR-99021), with or without stimulation with an anti-CD3 antibody (OKT3).
Figure 23 shows the results of measuring the cytokines (IFN-γ and IL-2) produced by stimulating CD4 single-positive helper T cells and CD8 single-positive killer T cells produced from hematopoietic stem cells derived from human iPS cells in the presence of a Wnt pathway activator (CHIR-99021) with PMA and ionomycin in the presence of momensin.

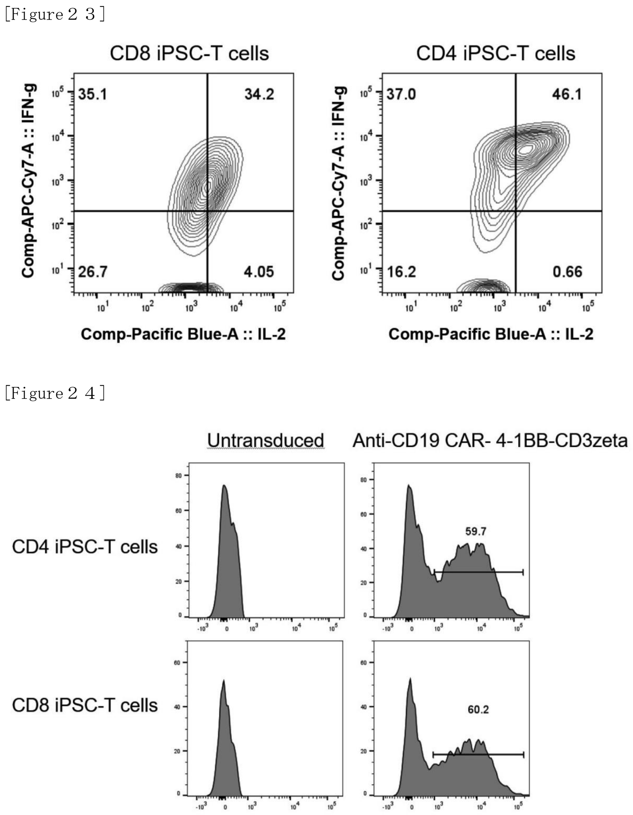

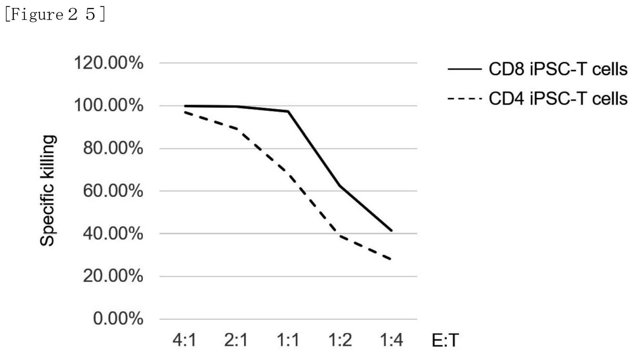

Figure 24 shows the results of flow cytometry analysis of anti-CD19 CAR expression in the resulting cells after gene transfer of anti-CD19 CAR (anti-CD19 CAR-4-1 BB-CD3zeta) using retroviral into CD4 single-positive helper T cells and CD8 single-positive killer T cells prepared from hematopoietic stem cells derived from human iPS cells in the presence of a Wnt pathway activator (CHIR-99021).Figure 25 shows the results of measurement of cytotoxic activity against the CD19-positive human pre-B cell leukemia cell line Nalm6 after transfection of anti-CD19 CAR (anti-CD19 CAR-4-1 BB-CD3zeta) into CD4 single-positive helper T cells and CD8 single-positive killer T cells prepared from hematopoietic stem cells derived from human iPS cells in the presence of a Wnt pathway activator (CHIR-99021).

### MODE FOR CARRYING OUT THE INVENTION [0019]

The present invention provides a method for producing a cell population containing CD4 single-positive, CD8-negative helper T cells. The first method of the present invention comprises the steps of: (1) culturing hematopoietic stem cells, differentiated from pluripotent stem cells, in the presence of a T cell differentiation factor; (2) culturing the cells obtained in step (1) in the presence of a T cell differentiation factor and a cell adhesion factor; and (3) culturing the cells obtained in step (2) in the presence of the cell adhesion factor but in the absence of the T cell differentiation factor. The second method of the present invention comprises the steps of: (1) culturing hematopoietic stem cells, differentiated from pluripotent stem cells, in the presence of a T cell differentiation inducing factor and a Wnt/β-catenin pathway activator; (2) culturing the cells obtained in step (1) in the presence of a cell adhesion factor, a Wnt pathway activator, and an anti-CD3 antibody, but in the absence of a T cell differentiation inducing factor; and (3) culturing the cells obtained in step (2) in the presence of a cell adhesion factor and a Wnt pathway activator, but in the absence of a T cell differentiation inducing factor and an anti-CD3 antibody.

### [Pluripotent Stem Cells]

In the present invention, pluripotent stem cells are stem cells that possess both the ability to proliferate almost indefinitely (self-renewal) and the ability to differentiate into almost all cell types that constitute an individual (multipotency). Pluripotent stem cells include, but are not limited to, embryonic stem cells (ES cells), induced pluripotent stem cells (iPS cells), embryonic germ stem cells (EG cells), multipotent germline stem cells (mGS cells), embryonic stem cells derived from cloned embryos obtained by nuclear transfer (ntES cells), and pluripotent stem cells isolated from fibroblasts and bone marrow mesenchymal cells (multi-lineage differentiating stress-enduring cells (Muse cells)). As pluripotent stem cells, iPS cells may be used, taking into consideration that they do not involve the destruction of embryos, eggs, etc., and have high differentiation potential.

### [Production of iPS Cells]

iPS cells are produced by reprogramming somatic cells from mammals. Mammals include humans, monkeys, pigs, dogs, cats, rats, and mice, with humans being preferred. The somatic cells are not particularly limited, but can be cells isolated from peripheral blood. In one embodiment of the present invention, the somatic cells are peripheral blood mononuclear cells (MBCs) or T cells depleted from B cells and T cells. Peripheral blood mononuclear cells depleted from B cells and T cells may be obtained by isolating monocytes from whole blood using a mononuclear cell separation solution, followed by removal of B cells and T cells using surface antigens expressed on B cells and T cells. Examples of mononuclear cell separation solutions include Lymphoprep^{®}. To remove B cells and T cells from mononuclear cells, antibodies against B cell surface antigens CD19, CD20, CD22, or the B cell receptor, and T cell surface antigens CD3, CD4, or CD8 may be used, for example, by flow cytometry or magnetic beads such as MACS^{®} beads. T cells can be isolated from peripheral blood by negative sorting to remove non-T cells such as monocytes, neutrophils, eosinophils, B cells, stem cells, dendritic cells, NK (natural killer) cells, and erythrocytes using specific antibodies against surface antigens (e.g., CD14, CD15, CD16, CD19, CD34, CD36, CD56, CD123, and CD234a (Glycophorin A)) present on these cells. T cells can also be isolated from peripheral blood mononuclear cells by positive selection.

Peripheral blood is a preferred source of T cells due to its minimal invasiveness, but is not limited to this. Other sources include cancer tissue, tumor tissue, or other tissues, umbilical cord blood, lymph, tissue fluid (interstitial fluid, intercellular fluid, and interstitial fluid), body cavity fluid (ascites, pleural effusion, pericardial fluid, cerebrospinal fluid, synovial fluid, and aqueous humor), nasal discharge, urinary pleural cavity, peritoneal cavity, cranial cavity, or spinal canal effusion (such as pleural effusion or ascites). Examples of cancer tissue or tumor tissue include tissue derived from ovarian cancer, hepatoblastoma, hepatocellular carcinoma, gastric cancer, esophageal cancer, pancreatic cancer, renal cell carcinoma, breast cancer, malignant melanoma, non-small cell lung cancer, cervical cancer, glioblastoma, prostate cancer, neuroblastoma, chronic lymphocytic leukemia, papillary thyroid cancer, colorectal cancer, head and neck cancer, brain tumor, multiple myeloma, or B-cell non-Hodgkin's lymphoma.

Methods for producing iPS cells are known in the art (see, for example, Takahashi K, Yamanaka S. Cell. 2006; 126:663-676; Takahashi K, et al. Cell. 2007; 131:861-872; Nakagawa M, et al. Nat Biotechnol. 2008; 26:101-106). In one embodiment of the present invention, iPS cells can be induced by introducing cell reprogramming factors into peripheral blood mononuclear cells or T cells from which cells and T cells have been depleted. Examples of cellular reprogramming factors include genes or gene products such as Oct3/4, Sox2, Sox1, Sox3, Sox15, Sox17, klf4, klf2, c-Myc, N-Myc, L-Myc, Nanog, Lin28, Fbx15, ERas, ECAT15-2, Tcl1, β-catenin, Lin28b, Sall1, Sall4, Esrrb, Nr5a2, Tbx3, and Glis1. These cell reprogramming factors may be used alone or in combination. Among these cell reprogramming factors, Oct3/4, Sox2, Klf4, and c-Myc (the so-called Yamanaka four factors) may be introduced into the peripheral blood mononuclear cells or T cells from the viewpoint of efficient establishment of iPS cells.

The method for introducing a cell reprogramming factor into peripheral blood mononuclear cells or T cells is not particularly limited, and any method known in the art can be used. For example, when introducing a gene encoding the cell reprogramming factor into peripheral blood mononuclear cells or T cells, the gene (e.g., cDNA) encoding the cell reprogramming factor can be inserted into an expression vector containing a promoter that functions in the peripheral blood mononuclear cells or T cells, and the expression vector can be introduced into the peripheral blood mononuclear cells or T cells by infection, lipofection, liposome, calcium phosphate coprecipitation, DEAE-dextran, microinjection, or electroporation. When the cell reprogramming factor is in the form of a protein and the protein is introduced into the peripheral blood mononuclear cells or T cells, examples of methods include a method using a protein introduction reagent, a method using a protein introduction domain fusion protein, electroporation, and microinjection. When the cell reprogramming factor is in the form of messenger RNA (mRNA) and the mRNA is introduced into the peripheral blood mononuclear cells or T cells, examples of the method include a method using an mRNA introduction reagent and a method of adding the mRNA to the culture medium.

Expression vectors used for gene transfer by infection include, for example, viral vectors such as lentivirus, retrovirus, adenovirus, adeno-associated virus, herpes virus, and Sendai virus, as well as animal cell expression plasmids. However, from the standpoint of being less susceptible to insertional mutagenesis, having high gene transfer efficiency, and allowing for a large number of copies of the transferred gene, Sendai virus may also be used to transfer the gene encoding the cellular reprogramming factor into the peripheral blood mononuclear cells or T cells.

Promoters used in expression vectors used to introduce the gene encoding the cellular reprogramming factor into the peripheral blood mononuclear cells or T cells include, for example, the SRα promoter, SV40 promoter, LTR promoter, CMV promoter, RSV promoter, HSV-TK promoter, and ubiquitin promoter. These promoters may be capable of controlling the expression of a gene inserted downstream of the promoter depending on the presence or absence of a drug such as tetracycline. In addition to the promoter, the expression vector may also include an enhancer, a poly(A) addition signal, a selectable marker gene (e.g., a neomycin resistance gene), an SV40 replication origin, and the like.

When using T cells to produce iPS cells, the T cells may be stimulated with anti-CD3 and anti-CD28 antibodies in the presence of interleukin-2 (IL-2) or stimulated with a desired antigen peptide to activate them before cell reprogramming. The stimulation can be performed by adding IL-2, anti-CD3, and anti-CD28 antibodies to the culture medium and culturing the T cells for a certain period of time. The anti-CD3 and anti-CD28 antibodies may be coated on cell culture beads or cell culture plates dispersed in the culture medium. Furthermore, stimulation with an antigen peptide can be performed by adding a peptide consisting of an amino acid sequence of at least nine residues that constitutes the desired antigen peptide recognized by the T cells to the culture medium and culturing the T cells for a certain period of time.

The culture medium used for culturing iPS cells is not particularly limited. A basal culture medium such as that used for culturing animal cells can be prepared by adding cytokines to maintain the undifferentiated state of iPS cells. Examples of basal culture medium include IMDM (Iscove's Modified Dulbecco's Medium), Medium 199, EMEM (Eagle's Minimum Essential Medium), αMEM (alpha Modified Eagle Minimum Essential Medium), DMEM (Dulbecco's modified Eagle's Medium), Ham's F12 medium, RPMI1640 medium, Fischer's medium, Neurobasal Medium (Life Technologies), StemFit^{®} AK03N (Ajinomoto Healthy Supply Co., Inc.), and mixtures thereof. The culture medium may contain serum or may be serum-free. An example of a cytokine is bFGF, and its concentration in the culture medium is, for example, 1-100 µg/mL.

iPS cells can be cultured using either adherent culture or suspension culture. When using suspension culture, iPS cells can be cultured in a cell culture dish until they reach 80% confluence, then dissociated into single cells and placed in suspension culture. Examples of methods for isolating iPS cells include physical isolation using a cell scraper, or isolation using a dissociation solution with protease activity, collagenase activity, or both protease and collagenase activity (e.g., Accutase^{®} and Accumax^{®}).

### [Induction of Differentiation from Pluripotent Stem Cells to Hematopoietic Stem Cells]

In the present invention, "hematopoietic stem cells" refer to cells that can differentiate into blood cell lineage cells such as lymphocytes, eosinophils, neutrophils, basophils, erythrocytes, and megakaryocytes. Hematopoietic stem cells and hematopoietic progenitor cells (HPCs) are not distinguished from each other and refer to the same cell type unless otherwise specified. Hematopoietic stem cells/progenitor cells can be recognized, for example, by their surface antigen CD34 positivity or by their double positivity for CD34 and CD43.

Inducing differentiation of pluripotent stem cells (iPS cells) into hematopoietic stem cells can be achieved by culturing iPS cells in a culture medium supplemented with vitamin C. Vitamin C refers to L-ascorbic acid and its derivatives. L-ascorbic acid derivatives are those that are converted to vitamin C via enzymatic reactions in vivo. Examples of L-ascorbic acid derivatives include vitamin C phosphate, ascorbic acid glucoside, ascorbyl ethyl, vitamin C ester, ascorbyl tetrahexyldecanoate, ascorbyl stearate, and ascorbyl-2-phosphate-6-palmitate. Examples of vitamin C phosphate include L-ascorbate phosphate salts such as sodium L-ascorbate phosphate or magnesium L-ascorbate phosphate. Vitamin C is contained in the culture medium at a concentration of 5 to 500 µg/mL, for example.

The culture medium used to induce differentiation of iPS cells into hematopoietic stem cells is not particularly limited, but can be prepared by adding vitamin C or other ingredients to a culture medium used for culturing animal cells as a basal culture medium. Examples of basal culture medium include IMDM (Iscove's Modified Dulbecco's Medium), Medium 199, EMEM (Eagle's Minimum Essential Medium), αMEM (alpha Modified Eagle Minimum Essential Medium), DMEM (Dulbecco's modified Eagle's Medium), Ham's F12 culture medium, RPMI1640 culture medium, Fischer's medium, Neurobasal Medium (Life Technologies), StemPro34 (Life Technologies), and mixtures of these. The culture medium may contain serum or may be serum-free. The basal culture medium may contain, as needed, one or more substances selected from, for example, albumin, insulin, transferrin, selenium, fatty acids, trace elements, 2-mercaptoethanol, thiolglycerol, monothiolglycerol, lipids, amino acids, L-glutamine, non-essential amino acids, vitamins, growth factors, low-molecular-weight compounds, antibiotics, antioxidants, pyruvic acid, buffers, inorganic salts, and cytokines.

The culture medium used to induce differentiation of iPS cells into hematopoietic stem cells may further contain cytokines selected from the group consisting of BMP4 (bone morphogenetic protein 4), VEGF (vascular endothelial growth factor), bFGF (basic fibroblast growth factor), SCF (stem cell factor), TPO (thrombopoietin), and FLT3L (FMS-like tyrosine kinase ligand). The concentrations of these cytokines are, for example, 1-100 ng/mL for BMP4, VEGF, bFGF, TPO, and FLT3L, and 10-100 ng/mL for SCF.

To induce differentiation of pluripotent stem cells into hematopoietic stem cells, they may be co-cultured with feeder cells such as C3H10T1/2 (Takayama N, et al. J Exp Med. 2010; 2817-2830) or heterologous stromal cells (Niwa A, et al. J Ce11 Physiol. 2009; 221:367-77).

Hematopoietic stem cells can be prepared from embryoid bodies formed by culturing iPS cells. Here, embryoid bodies are three-dimensional sac-like structures with internal spaces derived from iPS cells, formed from endothelial cell populations and other components, and containing hematopoietic stem cells.

### [T Cell Differentiation Inducing Factor]

In the present invention, a Notch ligand for a Notch molecule expressed on the cell membrane can be used as a T cell differentiation inducing factor. Mammals have Notch molecules, Notch 1 to 4, and binding between a Notch molecule and a Notch ligand induces Notch signaling, which is involved in regulating cell survival, proliferation, and differentiation (Artavanis-Tsakonas S, et al. Science. 1999; 284:770-776). Notch ligands are not particularly limited as long as they induce Notch signaling, but examples include Delta-Like-Protein (DLL) 1, 3, and 4, and Jagged 1 and 2. DLL1, DLL4, and Jagged 1 may also be used in the present invention. DLL4, an endogenous Notch1 ligand in mammals, particularly humans, may also be used in the present invention. The DLL4 may be a partial peptide of DLL4, as long as it induces Notch signaling. For example, a recombinant protein (DLL4-Fc) in which the Fc domain of human IgG1 is added to the C-terminus of the extracellular domain of human DLL4 (residue numbers 1 to 524 or residue numbers 27 to 529 (including mutations)) can be used.

### [Wnt/β-catenin pathway activators]

Signaling in the Wnt/β-catenin pathway is involved in numerous cellular processes and plays an important role in both animal development and regeneration (Gao J, et al. Cell Regeneration. 2021; 10:11). Wnt proteins primarily bind to Frizzled receptors, which are seven-transmembrane receptor proteins, to initiate signal transduction. Binding of Wnt proteins to the receptor induces the accumulation of β-catenin, which then functions as an intracellular mediator of Wnt signaling. Wnt/β-catenin pathway activators have the effect of enhancing signal transduction in this pathway. Wnt/β-catenin pathway activators are not particularly limited as long as they enhance signal transduction, and examples include inhibitors of glycogen synthase kinase 3 (GSK-3), a serine/threonine kinase. β-catenin is phosphorylated by GSK-3 and other proteins, then ubiquitinated and degraded by the proteasome; GSK-3 inhibitors inhibit the degradation of β-catenin.

GSK-3 has two isoforms, GSK-3α and GSK-3β. In the present invention, any of a GSK-3α inhibitor, a GSK-3β inhibitor, and a GSK-3α/β dual inhibitor may be used as long as it enhances signal transduction in the Wnt/β-catenin pathway. CHIR-99021 (6-[[2-[[4-(2,4-dichlorophenyl)-5-(5-methyl-1H-imidazol-2-yl)-2-pyrimidinyl]amino]ethyl]amino]-3-pyridinecarbonitrile) may also be used as a GSK-3 inhibitor. CHIR-99021 is also known as laduviglusib and is a compound with CAS number 252917-06-9. The concentration of the GSK-3 inhibitor added to the culture medium may be 0.01 to 100 µM.

### [Cell Adhesion Factor]

In the present invention, fibronectin or a fragment thereof can be used as a cell adhesion factor. Any region of fibronectin can be selected as the fibronectin fragment, as long as it retains the function required in the present invention. Fibronectin may be derived from human origin or may be a recombinant protein. In the present invention, Retronectin (registered trademark, Takara Bio Inc.) can be used as a cell adhesion factor. Retronectin is a recombinant protein that contains a cell adhesion domain (C-domain) that binds to α5β1 integrin (VLA-5), a heparin-binding domain (H-domain), and a CS-1 site that binds to α4β1 integrin (VLA-4) in human fibronectin.

During cell culture, the T cell differentiation inducing factor and the cell adhesion factor may be present coated on a cell culture substrate. "Present coated" refers to the T cell differentiation inducing factor and the cell adhesion factor being immobilized (coated) by adsorption or binding to the surface of the cell culture substrate. The coating can be achieved by diluting the T cell differentiation inducing factor and the cell adhesion factor to 1-100 µg/mL, for example, with phosphate buffer, Hank's balanced salt solution, or a serum-, Ca-, and Mg-free culture medium, and contacting the diluted solution with the cell culture substrate for a certain period of time. Commercially available cell culture beads (microcarriers) or cell culture plates can be used as the cell culture substrate.

### [Cell Culture]

The cell culture in steps (1) to (3) of the first and second methods of the present invention may be either adherent culture or suspension culture. For adherent culture, a cell culture plate can be used as the cell culture substrate on which the T cell differentiation inducing factor and the cell adhesion factor are coated, while for suspension culture, cell culture beads can be used. In the first method, step (1) is performed in the presence of the T cell differentiation inducing factor, step (2) is performed in the presence of the T cell differentiation inducing factor and the cell adhesion factor, and step (3) is performed in the presence of the cell adhesion factor but in the absence of the T cell differentiation inducing factor. In the second method, step (1) is performed in the presence of the T cell differentiation inducing factor and the Wnt/β-catenin pathway activator, step (2) is performed in the presence of the cell adhesion factor, the Wnt pathway activator, and an anti-CD3 antibody, and step (3) is performed in the presence of the cell adhesion factor and the Wnt pathway activator but in the absence of the T cell differentiation inducing factor and the anti-CD3 antibody. The anti-CD3 antibody used in step (2) of the second method may be, for example, clone number OKT3. The concentration of the anti-CD3 antibody added to the culture medium may be 0.01 to 100 µg/mL.

Examples of basal culture media used for cell culture in steps (1) to (3) of the first and second methods of the present invention include IMDM (Iscove's Modified Dulbecco's Medium), Medium 199, EMEM (Eagle's Minimum Essential Medium), αMEM (alpha Modified Eagle Minimum Essential Medium), DMEM (Dulbecco's modified Eagle's Medium), Ham's F12 culture medium, RPMI1640 culture medium, Fischer's medium, Neurobasal Medium (Life Technologies), and mixtures of these. Serum may be added to the culture medium, or it may be serum-free. Serum may be added, for example, in the form of 5-20% (v/v) fetal bovine serum. If necessary, the basal culture medium may contain one or more substances selected from the group consisting of albumin, insulin, transferrin, selenium, fatty acids, trace elements, 2-mercaptoethanol, thiolglycerol, monothiolglycerol, lipids, amino acids, L-glutamine, non-essential amino acids, vitamins, growth factors, low molecular weight compounds, antibiotics, antioxidants, pyruvic acid, buffers, inorganic salts, and cytokines.

In one embodiment of the first method of the present invention, the cell culture in steps (1) and (2) is carried out in the presence of interleukin-7 (IL-7) and FLT3L (FMS-like tyrosine kinase ligand). The concentrations of IL-7 and FLT3L in the culture medium are 0.1 to 50 ng/mL, and may also be 1 to 10 ng/mL.

In one embodiment of the first method of the present invention, the cell culture in steps (1) and (2) is carried out in the presence of IL-7, FLT3L, 2-phospho-L-ascorbic acid, and SDF-1α (stromal cell-derived factor 1). The concentrations of IL-7, FLT3L, and 2-phospho-L-ascorbic acid in the culture medium are 0.1 to 200 µg/mL, and may also be 1 to 100 µg/mL. The concentration of SDF-1α in the culture medium is 0.1 to 100 nM, and may further be 1 to 20 nM.

In one embodiment of the first method of the present invention, the cell culture in steps (1) and (2) is carried out in the presence of IL-7, FLT3L, 2-phospho-L-ascorbic acid, and a p38 inhibitor. The concentrations of IL-7, Flt3L, and 2-phospho-L-ascorbic acid in the culture medium are 0.1 to 200 µg/mL, and may even be 1 to 100 µg/mL. The concentration of the p38 inhibitor in the culture medium is 0.1 to 100 µM, and may even be 1 to 10 µM.

In one embodiment of the first method of the present invention, the cell culture in steps (1) and (2) is carried out in the presence of IL-7, FLT3L, a p38 inhibitor, and SDF-1α. The concentrations of IL-7 and Flt3L in the culture medium are 0.1 to 50 ng/mL, and may also be 1 to 10 ng/mL. The concentration of the p38 inhibitor in the culture medium is 0.1 to 100 µM, and may also be 1 to 10 µM. The concentration of SDF-1α in the culture medium is 0.1 to 100 nM, and may also be 1 to 20 nM.

In one embodiment of the first method of the present invention, the cell culture in steps (1) and (2) is carried out in the presence of IL-7, FLT3L, 2-phospho-L-ascorbic acid, SDF-1α, and a p38 inhibitor. The concentrations of IL-7, Flt3L, and 2-phospho-L-ascorbic acid in the culture medium are 0.1 to 200 µg/mL, and may even be 1 to 100 µg/mL. The concentration of SDF-1α in the culture medium is 0.1 to 100 nM, and may even be 1 to 20 nM. The concentration of the p38 inhibitor in the culture medium is 0.1 to 100 µM, and may even be 1 to 10 µM.

In one embodiment of the first method of the present invention, the cell culture in steps (1) and (2) is carried out in the presence of IL-7, FLT3L, 2-phospho-L-ascorbic acid, SDF-1α, a p38 inhibitor, and SCF (stem cell factor). The concentrations of IL-7, Flt3L, and 2-phospho-L-ascorbic acid in the culture medium are 0.1 to 200 µg/mL, and may be 1 to 100 µg/mL. The concentration of SDF-1α in the culture medium is 0.1 to 100 nM, and may be 1 to 20 nM. The concentration of the p38 inhibitor in the culture medium is 0.1 to 100 µM, and may be 1 to 10 µM. The concentration of SCF in the culture medium is 0.1 to 100 ng/mL, and may be 1 to 20 ng/mL.

In one embodiment of the first method of the present invention, the cell culture in step (3) is carried out in the presence of interleukin-7 (IL-7). The concentration of IL-7 in the culture medium is 0.1 to 50 ng/mL, and may also be 1 to 10 ng/mL.

In one embodiment of the first method of the present invention, the cell culture in step (3) is carried out in the presence of IL-7, 2-phospho-L-ascorbic acid, and SDF-1α (stromal cell-derived factor 1). The concentrations of IL-7 and 2-phospho-L-ascorbic acid in the culture medium are 0.1 to 200 µg/mL, and may even be 1 to 100 µg/mL. The concentration of SDF-1α in the culture medium is 0.1 to 100 nM, and may even be 1 to 20 nM.

In one embodiment of the first method of the present invention, the cell culture in step (3) is carried out in the presence of IL-7, 2-phospho-L-ascorbic acid, and a p38 inhibitor. The concentrations of IL-7 and 2-phospho-L-ascorbic acid in the culture medium are 0.1 to 200 µg/mL, and may even be 1 to 100 µg/mL. The concentration of the p38 inhibitor in the culture medium is 0.1 to 100 µM, and may even be 1 to 10 µM.

In one embodiment of the first method of the present invention, the cell culture in step (3) is carried out in the presence of IL-7, a p38 inhibitor, and SDF-1α. The concentration of IL-7 in the culture medium is 0.1 to 50 ng/mL, and may also be 1 to 10 ng/mL. The concentration of the p38 inhibitor in the culture medium is 0.1 to 100 µM, and may also be 1 to 10 µM. The concentration of SDF-1α in the culture medium is 0.1 to 100 nM, and may also be 1 to 20 nM.

In one embodiment of the first method of the present invention, the cell culture in step (3) is carried out in the presence of IL-7, 2-phospho-L-ascorbic acid, SDF-1α, and a p38 inhibitor. The concentrations of IL-7 and 2-phospho-L-ascorbic acid in the culture medium are 0.1 to 200 µg/mL, and may even be 1 to 100 µg/mL. The concentration of SDF-1α in the culture medium is 0.1 to 100 nM, and may even be 1 to 20 nM. The concentration of the p38 inhibitor in the culture medium is 0.1 to 100 µM, and may even be 1 to 10 µM.

In one embodiment of the first method of the present invention, the cell culture in step (3) is carried out in the presence of IL-7, 2-phospho-L-ascorbic acid, SDF-1α, a p38 inhibitor, and SCF (stem cell factor). The concentrations of IL-7 and 2-phospho-L-ascorbic acid in the culture medium are 0.1 to 200 µg/mL, and may also be 1 to 100 µg/mL. The concentration of SDF-1α in the culture medium is 0.1 to 100 nM, and may also be 1 to 20 nM. The concentration of the p38 inhibitor in the culture medium is 0.1 to 100 µM, and may also be 1 to 10 µM. The concentration of SCF in the culture medium is 0.1 to 100 ng/mL, and may also be 1 to 20 ng/mL.

In one embodiment of the second method of the present invention, the cell culture in step (1) is carried out in the presence of IL-7, FLT3L, 2-phospho-L-ascorbic acid, SDF-1α, a p38 inhibitor, SCF, and TPO (thrombopoietin). The concentrations of IL-7, FLT3L, SCF, and TPO in the culture medium are 1 to 500 ng/mL, and may be 10 to 100 ng/mL. The concentration of 2-phospho-L-ascorbic acid in the culture medium is 1 to 1000 µg/mL, and may even be 5 to 100 µg/mL. The concentration of SDF-1α in the culture medium is 0.1 to 500 nM, and may even be 1 to 100 nM. The concentration of the p38 inhibitor in the culture medium is 0.1 to 200 µM, and may even be 1 to 50 µM.

In one embodiment of the second method of the present invention, the cell culture in steps (2) and (3) is carried out in the presence of IL-7, 2-phospho-L-ascorbic acid, SDF-1α, and a p38 inhibitor. The concentration of IL-7 in the culture medium is 1 to 500 ng/mL, and may be 10 to 100 ng/mL. The concentration of 2-phospho-L-ascorbic acid in the culture medium is 1 to 1000 µg/mL, and may even be 5 to 100 µg/mL. The concentration of SDF-1α in the culture medium is 0.01 to 200 nM, and may even be 1 to 50 nM. The concentration of the p38 inhibitor in the culture medium is 0.1 to 100 mM, and may even be 1 to 50 mM.

In the culture medium used in steps (1) to (3) of the first and second methods of the present invention, isoforms such as SDF-1β, SDF-1γ, SDF-1δ, SDF-1ε, or SDF-1ϕ may be used in place of SDF-1α, or mixtures of these in any proportions may be used.

The p38 inhibitor is defined as a substance that inhibits the function of p38 protein (p38 MAP kinase). Examples of p38 inhibitors include chemical inhibitors of p38. Examples of chemical inhibitors of p38 include, but are not limited to, SB203580 (4-(4-fluorophenyl)-2-(4-methylsulfonylphenyl)-5-(4-pyridyl)-1H-imidazole) and derivatives thereof, SB202190 (4-(4-fluorophenyl)-2-(4-hydroxyphenyl)-5-(4-pyridyl)-1H-imidazole) and derivatives thereof, SB239063 (trans-4-[4-(4-fluorophenyl)-5-(2-methoxy-4-pyrimidinyl)-1H-imidazol-1-yl]cyclohexanol) and derivatives thereof, SB220025 and derivatives thereof, PD169316, RPR200765A, AMG-548, BIRB-796, SCI0-469, SCI0-323, VX-702 and FR167653.

The cell culture temperature in steps (1) to (3) of the first and second methods of the present invention is not particularly limited as long as it does not inhibit cell growth and survival, but can be about 37°C to about 42°C, and may also be about 37°C to about 39°C.

The culture period in step (1) of the first and second methods of the present invention can be determined by confirming the formation of T cell precursors. In this invention, the term T cell precursor refers to CD3, CD5, and CD1a positive cells that are fully committed to the T cell lineage. The T cell precursors include not only CD4/CD8 double-positive immature T cells, but also CD4 single-positive immature T cells. These cell surface antigens can be detected by flow cytometry using fluorescently labeled antibodies against the cell surface antigens.

In one embodiment of the present invention, after confirming the appearance of CD3-, CD1a-, and CD5-positive T precursor cells among the cells obtained in step (1), the next step (2) may be carried out. That is, the step of confirming that the cells obtained in step (1) contain CD3-, CD1a-, and CD5-positive T precursor cells may be a step of determining whether to proceed from step (1) to step (2). The culture period in step (1) is not particularly limited as long as CD3- and CD5-positive cells are obtained, and may be, for example, 10 to 30 days.

The culture period in step (2) of the first method of the present invention can be determined by confirming the formation of CD3-positive and CD4/CD8 double-positive immature T cells. In one embodiment of the present invention, after confirming the presence of CD3-positive and CD4/CD8 double-positive immature T cells in the culture of step (2), the method may proceed to the next step (3). In other words, the step of confirming that the cells obtained in step (2) contain CD3-positive and CD4/CD8 double-positive immature T cells can be a step of determining whether to proceed from step (2) to step (3). In one embodiment of the present invention, after confirming that the cells obtained in step (2) are CD3-positive and CD4/CD8 double-positive immature T cells and further contain CD5- and CD1a-positive cells, the next step (3) may be proceeded to. That is, the step of confirming that the cells obtained in step (2) are CD3-positive and CD4/CD8 double-positive immature T cells and further contain CD5- and CD1a-positive cells may be a step of determining whether to proceed from step (2) to step (3). The culture period in step (2) is not particularly limited as long as CD3-positive and CD4/CD8 double-positive cells are obtained, and may be, for example, 3 to 10 days.

The culture period in step (3) of the first and second methods of the present invention can be determined by confirming the formation of mature helper T cells that are CD1a and CD8 negative, and CD5 positive and CD4 single positive, or a mixture of the mature helper T cells and mature killer T cells that are CD5 and CD8 positive and CD1a and CD4 negative. By confirming the formation of mature helper T cells that are CD1a and CD8 negative, and CD5 positive and CD4 single positive, or a mixture of the mature helper T cells and mature killer T cells that are CD5 and CD8 positive and CD1a and CD4 negative, among the cells obtained in step (3), it can be determined that a cell population containing helper T cells that are CD4 single positive and CD8 negative has been obtained. In the methods of the present invention, mature helper T cells and mature killer T cells may be formed simultaneously. The culture period in step (3) is not particularly limited as long as mature helper T cells that are CD1a and CD8 negative, CD5 positive, and CD4 single positive can be obtained, but may be, for example, 2 to 20 days.

In one embodiment of the first and second methods of the present invention, the cells obtained in step (3) may be confirmed to contain T cells that highly express CD28. The term "high expression" means that the CD28 expression level is equivalent to that of primary T cells obtained from peripheral blood. CD28 is a costimulatory receptor essential for T cell activation, and therefore serves as an indicator that the obtained cells are functional helper T cells. In other words, the step of confirming that the cells obtained in step (3) contain T cells that highly express CD28 may be a step of determining whether the helper T cells obtained by the method of the present invention are functional helper T cells. In one embodiment of the present invention, the cells obtained in step (3) may be confirmed to exhibit a CD28 expression level equivalent to that of mature T cells present in human peripheral blood. The term "equivalent" means that, in flow cytometry analysis, the CD28 expression level is similar to that of a naive T cell fraction that expresses CD28 most highly, to the extent that it is difficult to distinguish between them.

In one embodiment of the first method of the present invention, the cell culture in steps (1) and (2) is carried out while maintaining the cell density at subconfluence. Confluence refers to a state in which the cell adhesion surface of the cell culture substrate is completely covered with cultured cells, leaving no room for the cells to grow as a monolayer. In the present invention, "subconfluence" refers to a state in which 50-90% of the adhesion surface of the culture vessel is occupied by cultured cells, and the occupancy rate of the adhesion surface of the culture vessel by cultured cells may be 60-80%.

The first and second methods of the present invention, which include steps (1) to (3), can produce a cell population containing CD4 single-positive, CD8-negative helper T cells. The term "cell population" refers to cells that are classified as CD4 single-positive/CD8-negative helper T cells, but may also include a variety of cells, such as cells with different cell surface antigen expression patterns and CD4 negative/CD8 positive killer T cells. The resulting CD4 positive/CD8 negative helper T cells and CD4 negative/CD8 positive killer T cells may be isolated and used individually or as a mixture. Isolation can be performed using methods well known in the art. For example, labeling with antibodies against CD4, CD8, CD3, and/or CD45 and isolating using a flow cytometer, or purification using an affinity column on which the desired antigen is immobilized, are examples of such methods.

### [Introduction of cell surface molecules reactive to tumor-associated antigens into cells]

A nucleic acid encoding a chimeric antigen receptor (CAR) or T cell receptor (TCR) molecule reactive to a tumor-associated antigen can be introduced extracellularly into the pluripotent stem cells or iPS cells, the T cell precursors, the hematopoietic stem cells, the immature T cells, or the mature T cells.

When introducing a TCR, cDNA encoding the TCR α and β chains or cDNA encoding the γ and δ chains can be prepared and incorporated into an expression vector. These cDNAs can also be incorporated into viral or non-viral vectors (transposon vectors), for example, using the Gibson assembly system. Specifically, downstream of a ubiquitin promoter, a gene containing cDNA encoding the TCR α and TCR β chains linked via a T2A sequence can be ligated. Further downstream, a marker gene such as EGFR with its ligand-binding site and intracellular domain removed (EGFRt, truncated EGFR) or CD19 lacking the intracellular domain can be linked to an IRES (internal ribosome entry site) sequence, and this construct can be incorporated into a viral or non-viral vector. The cDNA encoding the TCR α chain and the cDNA encoding the β chain can also be incorporated into separate expression vectors.

When introducing a CAR, cDNA encoding the CAR can be prepared and incorporated into an expression vector. For example, total RNA is extracted from the lymph nodes of an animal immunized with the desired tumor-associated antigen and used as a template to synthesize cDNA; the light and heavy chains of the variable regions of a monoclonal antibody against the tumor-associated antigen are amplified separately, linked using a flexible linker, and amplified by assembly PCR; a CAR construct can be prepared by linking the sequences encoding the fused light and heavy chains to a sequence encoding the intracellular domain of the CAR molecule via a sequence encoding the transmembrane domain of the CAR molecule. To confirm expression of the introduced gene, EGFR (EGFRt) minus the ligand-binding site and intracellular domain may be linked to this CAR construct via a T2A sequence. The flexible linker that connects the light and heavy chains of the variable regions of a monoclonal antibody is a linker peptide of approximately 5 to 20 amino acid residues and is known in the art (see, for example, Ueda T, et al. Cancer Sci. 2020; 111:1478-1490. Kawasaki Tomomi et al., SCEJ 72nd Annual Meeting (Kyoto, 2007) H209.).

The antigen-binding domain to be incorporated into the CAR may be produced using phage display without immunizing an animal with the antigen. For example, antibodies that specifically bind to a desired tumor-associated antigen may be screened from a phage display antibody library expressing the Fab regions of a large number of human antibodies.

A TCR or CAR reactive to a tumor-associated antigen may be introduced into the cells alone, or a TCR and a CAR may be introduced simultaneously. When a TCR and a CAR are introduced simultaneously, the expression vectors may be the same or different. When a TCR and a CAR are introduced simultaneously, the TCR and the CAR may be reactive to the same tumor-associated antigen, or may be reactive to different tumor-associated antigens.

Viral and non-viral vectors can be used as expression vectors. Examples of viral vectors include lentivirus, retrovirus, adenovirus, adeno-associated virus, herpes virus, and Sendai virus, as well as animal cell expression plasmids. Lentivirus or retrovirus is preferred. When retrovirus or lentivirus infection is used, spin-infection methods may also be used. Non-viral vectors include the piggyBac (registered trademark) vector, which is a transposon vector. Genome editing techniques may be used to replace the endogenous TCR of iPS cells with a TCR or CAR introduced from outside the cells. Examples of such genome editing techniques include the CRISPR/Cas9 method, the CRISPR/MAD method, and the CRISPR/CAS3 method. Methods for introducing non-viral vector genes or methods for introducing guide RNA and donor DNA for genome editing include lipofection, liposome, calcium phosphate coprecipitation, DEAE-dextran, microinjection, and electroporation. Gene transfer of the TCR and/or CAR may be performed into the TCR locus or other loci (e.g., the β2-microglobulin locus). When modification or disruption of existing genes, including the TCR, by the introduced gene is not desired, safe harbor loci may be used. Examples of such safe regions include the AAVS1 (Adeno-associated virus integration site 1) region in the human genome. Examples of methods for introducing a gene by targeting the safe region include the CRISPR/Cas9 method and the TALEN method.

"Tumor-associated antigens" are antigens that are expressed specifically or non-specifically in tumors, including antigens derived from proteins overexpressed in tumor cells and their mutants, antigens derived from tumor viruses, certain differentiation antigens, and novel tumor-associated antigens (neoantigens) resulting from genetic mutations and splice aberrations. Protein antigens may also be fragmented peptides (peptide fragments). Antigens expressed specifically or non-specifically in tumors include, but are not limited to, WT1, GPC3, BCMA, XAGE1, MUC1, MUC5A1, MUC6, EGFRvIII, HER-2/neu, MAGE-A1, MAGE-A3, telomerase, PRAME, SSX2/4, PSCA, CTLA-4, gp100, GD2, GD3, fucosyl GM1, GM3, sLe(a), glycolipid F77, mesothelin, PD-L1, trp1, trp2, CD19, CD20, CD22, ROR1, CD33, c-Met, p53 without gene mutation, p53 with gene mutation, p53 mutant, NY-ESO-1, PSMA, ETV6-AML, CEA, PSA, AFP, hTERT, EpCAM, ALK, androgen receptor, EphA2, CYP1B1, OY-TES-1, MAD-CT-2, MelanA/MART1, survivin, Ras, Ras mutants, EGR, bcr-abl, XBP-1, neoantigens due to gene mutations, and neoantigens due to splice aberrations. Viral antigens include, but are not limited to, HBV, HBs, HPV, EBV, LMP1, EBV, LMP2, EBNA, HPV-E1, HPV-E2, HPV-E6, HPV-E7, HTLV-1 Tax, and HBZ.

### [Pharmaceuticals Containing Cell Populations Produced by the Methods of the Present Invention]

Pharmaceuticals containing cell populations produced by the methods of the present invention can be used as agents for preventing or treating cancer in mammals. The pharmaceuticals of the present invention may be produced by methods conventional in the pharmaceutical technology field. The pharmaceuticals of the present invention may contain pharmaceutically acceptable additives. Examples of such additives include cell culture medium, physiological saline, and appropriate buffer solutions (e.g., phosphate buffer solutions).

The pharmaceutical of the present invention can be manufactured by suspending the cells of the present invention in physiological saline or an appropriate buffer solution (e.g., phosphate buffer). To achieve the desired therapeutic effect, a single dose may contain, for example, 1 x 10⁷ or more, 1 x 10⁸ or more, or 1 x 10⁹ or more cells. The cell content can be adjusted taking into account the gender, age, weight, condition of the affected area, and cell condition of the recipient. In addition to the cells of the present invention, the pharmaceutical of the present invention may contain dimethyl sulfoxide (DMSO) and serum albumin for cell protection. It may also contain antibiotics to prevent bacterial contamination. It may also contain vitamins and cytokines for promoting cell activation and differentiation. The pharmaceutical of the present invention may also contain other pharmaceutically acceptable ingredients (e.g., carriers, excipients, disintegrants, buffers, emulsifiers, suspending agents, soothing agents, stabilizers, preservatives, antiseptics, physiological saline, etc.).

Pharmaceuticals containing the cells of the present invention can be cryopreserved. When cryopreserved, the storage temperature is not particularly limited as long as it is suitable for cell storage. Examples include -20°C, -80°C, and -120 to -196°C, with -150°C or below being preferred. When cryopreserved, the cells may be stored in an appropriate container such as a cryovial or cryobag.

The pharmaceutical of the present invention is used for the prevention or treatment of cancer. Examples of cancer include, but are not limited to, ovarian cancer, hepatoblastoma, hepatocellular carcinoma, gastric cancer, esophageal cancer, pancreatic cancer, renal cell carcinoma, breast cancer, malignant melanoma, non-small cell lung cancer, cervical cancer, glioblastoma, prostate cancer, neuroblastoma, chronic lymphocytic leukemia, papillary thyroid cancer, colorectal cancer, head and neck cancer, brain tumor, multiple myeloma, and B-cell non-Hodgkin's lymphoma.

The cells of the present invention are capable of killing cells expressing tumor-associated antigens and can therefore be used as a killing agent for cells expressing tumor-associated antigens. This killing agent can be manufactured and used in the same manner as the pharmaceutical.

The present invention will be described below using examples, but the present invention is not limited to these examples.

### Example 1

### [Production of CD4 Single-Positive Helper T Cells from iPS Cells (Method 1)]

iPS cells were produced from CD3-positive T cells isolated from human peripheral blood using the method described in Non-Patent Document 3. A pan T Cell Isolation Kit (Miltenyi Biotec, Catalog No. 130-096-535) was used to isolate the T cells.

### (Day 0)

150 µL of DLL4-Fc solution (Sino bio, Catalog No. 10171-H02H) diluted to 5 µg/mL in phosphate-buffered saline (PBS) was added to each well of a 48-well cell culture plate (Corning, Catalog No. 3548) and allowed to stand overnight at 4°C to coat the plate with human DLL4-Fc. DLL4-Fc is a recombinant protein in which the Fc domain of human IgG1 is attached to the C-terminus of the extracellular domain (residues 1-524) of human DLL4 (Delta-Like Protein 4), and was used as a T cell differentiation inducing factor.

### Days 1 to 14: Step (1)

### (Day 1)

Embryoid bodies formed by 11 days of suspension culture of iPS cells were collected and dispersed into single cells by six cycles of aspirating and discharging using a syringe equipped with a 21-gauge needle. Cell debris was then removed using a cell strainer. The resulting cells were suspended in T cell differentiation medium and adjusted to a cell concentration of 4 x 10⁴ cells/mL. The DLL4-Fc-coated cell culture plate was washed once with 1 mL/well of PBS, and 500 µL/well of T cell differentiation medium was added. 500 µL/wellof the cell suspension (4 x 10⁴ cells/mL) was then added. The final culture medium volume was 1 mL/well. The cells added to the cell culture plate were cultured at 37°C in a 5% CO₂ environment, with half of the culture medium being replaced with fresh T cell differentiation medium every 2 to 3 days. During culture, the cell density was maintained at 70 to 80% confluence or less.

As the T cell differentiation culture medium, the following components were used: 15% fetal bovine serum (Nakarai Tesque, catalog number: 01863-48), PSG (penicillin-streptomycin-L-glutamine solution, 100×, Sigma-Aldrich, catalog number: G1146-100ML), ITS (insulin-transferrin-sodium selenite) supplement (100X, Invitrogen, catalog number: 41400045), 2-phospho-L-ascorbic acid (final concentration 50 µg/mL, Sigma-Aldrich, catalog number: A8960-5G), human recombinant IL-7 (final concentration 5 ng/mL, PeproTech, catalog number: 200-07), human recombinant Flt3L (final concentration 5 ng/mL, PeproTech, catalog number: 300-19), human recombinant SCF (final concentration 10 ng/mL, R&D Systems, catalog number: 255-SC), human recombinant SDF-1α (final concentration 10 nM, PeproTech, catalog number: 300-28A), and the p38 inhibitor SB203580 (final concentration 10 µM, Tocris Bioscience, catalog number: 1202), mixed in α-MEM (alpha Modified Eagle Minimum Essential Medium, Gibco, catalog number: 12571-063).

### (Day 7)

A new 48-well cell culture plate was coated with DLL4-Fc in the same manner as on Day 0.

### (Day 8)

The new DLL4-Fc-coated 48-well cell culture plate was washed once with 1 mL/well of PBS, and then 500 µL/well of the T cell differentiation medium was added. 500 µL/well of a culture medium containing the cultured cells at 1-2 x 10⁵ cells/mL was added. The cells added to the new cell culture plate were cultured at 37°C in a 5% CO₂ environment, with half of the culture medium replaced with fresh T cell differentiation medium every 2-3 days. During culture, the cell density was maintained at approximately 70-80% confluence or less.

### (Day 14)

The obtained cells were analyzed by flow cytometry, confirming that the cells differentiated from iPS cells contained T cell precursors that were both CD3 and CD5 positive.

To each well of a new 48-well cell culture plate, 150 µL of PBS containing 5 µg/mL human DLL4-Fc and RetroNectin (RetroNectin, registered trademark, Takara Bio) was added and left to stand overnight at 4°C to coat the cell culture plate with human DLL4-Fc and RetroNectin. RetroNectin was used as a cell adhesion factor.

### Days 15 to 21: Step (2)

### (Day 15)

The cell culture plate coated with DLL4-Fc and retronectin was washed once with 1 mL/well of PBS, and the T cell differentiation medium was added at 500 µL/well. A cell suspension containing the CD3- and CD5-positive T cell precursors (2-4 x 10⁵ cells/mL) was added at 500 µL/well. These cells were cultured at 37°C in a 5% CO₂ environment, with half of the culture medium replaced with fresh T cell differentiation medium every 2-3 days. During culture, the cell density was maintained at approximately 70-80% confluence.

### (Day 21)

The obtained cells were analyzed by flow cytometry, and it was confirmed that the cells differentiated from T progenitor cells contained CD3-positive, CD4/CD8-double-positive, and CD5- and CD1a-positive T cells.

To each well of a new 48-well cell culture plate, 150 µL of RetroNectin solution diluted to 5 µg/mL with PBS was added and the plate was left to stand overnight at 4°C to coat the plate with RetroNectin.

### Days 22 to 35: Step (3)

### (Day 22)

A culture medium (Flt3L-free T cell differentiation medium) was prepared by removing human recombinant Flt3L from the T cell differentiation medium. After washing the new retronectin-coated cell culture plate once with 1 mL/well of PBS, 500 µL/well of the Flt3L-free T cell differentiation medium was added. To this was added 500 µL/well of the Flt3L-free T cell differentiation medium containing the T cells cultured and differentiated in the cell culture plate up to this point at 8 x 10⁵ cells/mL. The cells were then cultured at 37°C in a 5% CO₂ environment, with half of the culture medium replaced with fresh Flt3L-free T cell differentiation medium every 2 to 3 days.

### (Day 28)

A new 48-well cell culture plate was coated with RetroNectin in the same manner as on Day 21.

### (Day 29)

The RetroNectin-coated new 48-well cell culture plate was washed once with 1 mL/well of PBS, and then 500 µL/well of the Flt3L-free T cell differentiation medium was added. The culture medium in the wells containing the T cells cultured in the cell culture plate was then reduced to 500 µL, and all of the cells contained in each well were transferred, along with the culture medium, to the wells of the new 48-well cell culture plate. The plate was then further cultured at 37°C in a 5% CO₂ environment.

### (Day 35)

Flow cytometry analysis of the obtained cells confirmed the presence of mature helper T cells that were CD4 single positive, CD8 negative, CD5 and CD3 positive, and CD1a negative. Furthermore, the presence of mature killer T cells that were CD8 single positive, CD4 negative, CD5 and CD3 positive, and CD1a negative was also confirmed.

### Example 2

### [Effect of Coating Cell Culture Plates with DLL4-Fc and Retronectin on the Production of CD4 Single-Positive Helper T Cells from iPS Cells (1)]

The effect of coating cell culture plates with DLL4-Fc and Retronectin on the production of CD4 single-positive helper T cells from iPS cells was examined in each of steps (1) (days 1-14), (2) (days 15-21), and (3) (days 22-35) shown in Example 1. For this study, cells were cultured using various combinations of coatings on the cell culture plates in each step. The coating of DLL4-Fc and Retronectin, iPS cell culture, and culture conditions were similar to those in Example 1.

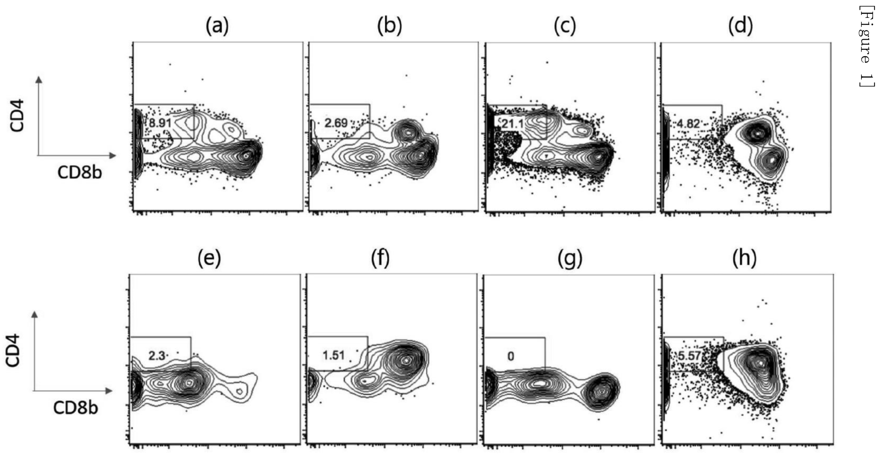

Table 1 shows the combinations of DLL4-Fc and Retronectin coating of cell culture plates in each step. In Table 1, coating combination (c) represents the coating conditions used in Example 1. Specifically, for coating combination (c), cells were cultured using cell culture plates coated with DLL4-Fc alone in step (1) (days 1 to 14), cell culture plates coated with DLL4-Fc and Retronectin in step (2) (days 15 to 21), and cell culture plates coated with Retronectin alone in step (3) (days 22 to 35). Figure 1 shows the results of flow cytometry analysis of CD4 single-positive helper T cell production for coating combinations (a) to (h). The values shown in each dot plot in Figure 1 indicate the percentage of CD3 and CD5 positive/CD1a negative/CD4 single-positive mature helper T cells in the cell population obtained on day 35 of the culture schedule. The proportions are also shown in Table 1.

| **Process and culture schedule** | | **Coating combination** | | | | | | | |
|---|---|---|---|---|---|---|---|---|---|
| | | (a) | (b) | (c) | (d) | (e) | (f) | (g) | (h) |
| | **Process (1) Days 1 to 14** | D | D | D | D | D + R | D + R | D + R | D + R |
| | **Process (2) Days 15 to 21** | D | D | D + R | D + R | D | D | D + R | D + R |
| | **Process (3) Days 22 to 35** | R | D + R | R | D + R | R | D + R | R | D + R |
| **Percentage of existence of CD4SP iPSC-T cell (%)** | | 8.91 | 2.69 | 21. 1 | 4.82 | 2.3 | 1.51 | 0 | 5.57 |

(Abbreviations used in the table)
D: DLL4-Fc
R: Retronectin
CD4SP iPSC-T cells: CD4 single-positive helper T cells derived from iPS cells

Figure 1 and Table 1 demonstrate that the presence or absence of DLL4-Fc (a T cell differentiation inducing factor) and retronectin (a cell adhesion factor) in each step is important for producing CD4 single-positive helper T cells from iPS cells. Specifically, by applying a combination of DLL4-Fc single-coating in step (1) (days 1 to 14), DLL4-Fc and retronectin double-coating in step (2) (days 15 to 21), and retronectin single-coating in step (3) (days 22 to 35) (coating combination (c)), the proportion of CD4 single-positive helper T cells in total cells was found to be significantly higher than with other combinations. Meanwhile, as shown in Figure 1, the presence or absence of coating on the cell culture plate was not important for producing CD8 single-positive killer T cells from iPS cells.

### Example 3

### [Effect of Coating Cell Culture Plates with DLL4-Fc and Retronectin on the Production of CD4 Single-Positive Helper T Cells from iPS Cells (2)]

In step (3) (days 22 to 35) shown in Example 1, the effect of coating cell culture plates with DLL4-Fc and Retronectin on the production of CD4 Single-Positive Helper T Cells from iPS Cells was examined. The coating of DLL4-Fc and Retronectin, iPS cell culture, and culture conditions were similar to those in Example 1.

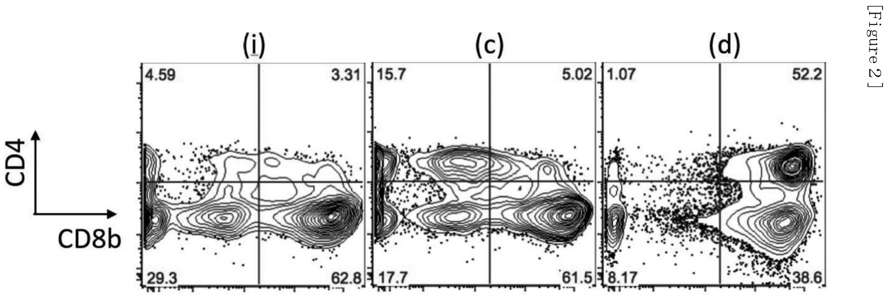

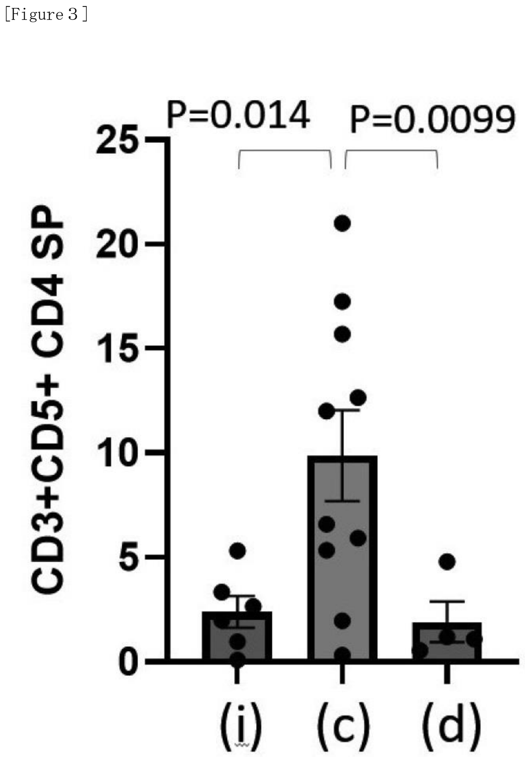

Table 2 shows the combinations of DLL4-Fc and RetroNectin coating of the cell culture plate in step (3). The coating conditions were the same for steps (1) and (2). In Table 2, "coating combination (c)" refers to the coating conditions used in Example 1. Figure 2 shows the results of flow cytometry analysis of CD4 single-positive helper T cells produced with coating combinations (i), (c), and (d). The results showed that, in step (3), "coating combination (c)," in which the cell culture plate was coated with RetroNectin, produced a higher amount of CD4 single-positive helper T cells than "coating combination (i)," in which the cell culture plate was not coated, and "coating combination (d)," in which the cell culture plate was double-coated with DLL4-Fc and RetroNectin. Furthermore, the percentages (%) of CD3 and CD5 positive/CD1a negative/CD4 single positive mature helper T cells in the cell populations obtained on day 35 of the culture schedule were compared among coating combinations (i), (c), and (d). The results (mean ± standard error) are shown in Table 2 and Figure 3. The n values in Table 2 indicate the number of independent experiments. Statistical analysis was performed using a paired t-test. Coating combination (c) showed a statistically significantly higher percentage of CD3 and CD5 positive/CD1a negative/CD4 single positive mature helper T cells compared to coating combinations (i) and (d) (Figure 3). This indicates that RetroNectin alone is effective in producing CD4 single positive helper T cells in step (3).

**(Table 2)**

| **Process and culture schedule** | **Coating combination** | | |
|---|---|---|---|
| | (i) | (c) | (d) |
| **Process (1): Days 1 to 14** | D | D | D |
| **Process (2): Days 15 to 21** | D + R | D + R | D + R |
| **Process (3): Days to 35** | - | R | D + R |
| **Percentage of existence of CD4SP iPSC-T cell (%)** | 2.39±0.75 (n=6) | 9.88±2.17 (n=10) | 1.92±0.97 (n=4) |

(Abbreviations used in the table)
D: DLL4-Fc
R: Retronectin
-: No coating
CD4SP iPSC-T cells: CD4 single-positive helper T cells derived from iPS cells

### Example 4

### [Effect of Coating Cell Culture Plates with DLL4-Fc and Retronectin on the Production of CD4 Single-Positive Helper T Cells from iPS Cells (3)]

The results obtained in Example 3 demonstrated that coating culture plates with Retronectin alone in step (3) resulted in a higher production of CD4 single-positive helper T cells. Therefore, the effect of coating cell culture plates with both DLL4-Fc and Retronectin in step (1) (days 1-14) and step (2) (days 15-21) on the production of CD4 single-positive helper T cells from iPS cells was examined. Specifically, the effect of coating Retronectin in addition to DLL4-Fc in steps (1) and (2) was examined. The coating of DLL4-Fc and Retronectin, iPS cell culture, and culture conditions were similar to those in Example 1.

Table 3 shows the combinations of DLL4-Fc and RetroNectin coating of cell culture plates in steps (1) and (2). The coating conditions for step (3) were the same. In Table 3, "coating combination (c)" refers to the coating conditions used in Example 1.

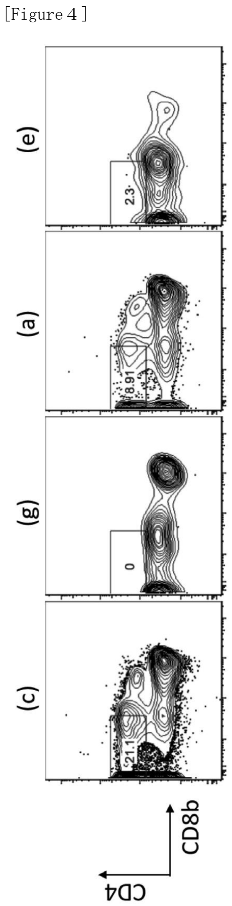

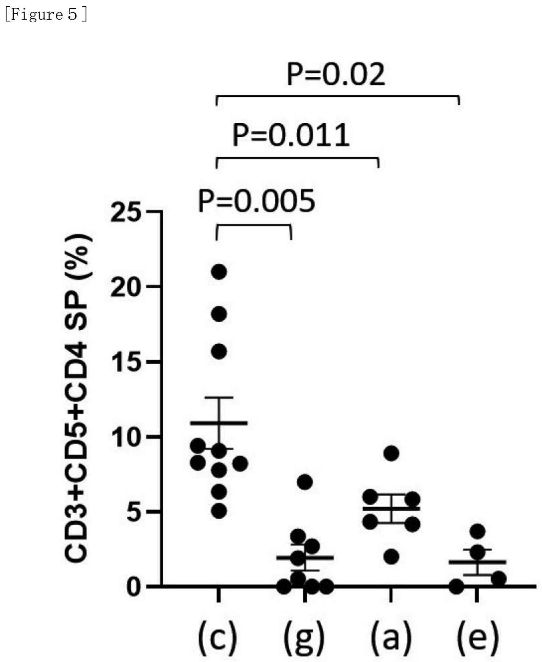

Figure 4 shows the results of flow cytometry analysis of CD4 single-positive helper T cells produced by coating combinations (a), (c), (e), and (g). The results showed that, with respect to steps (1) and (2), "coating combination (c)," in which the cell culture plate was additionally coated with RetroNectin only in step (2), produced a higher amount of CD4 single-positive helper T cells than "coating combination (a)," in which the cell culture plate was not coated with RetroNectin; "coating combination (e)," in which RetroNectin was additionally coated only in step (1); and "coating combination (g)," in which RetroNectin was additionally coated in steps (1) and (2). Furthermore, the percentages (%) of CD3 and CD5 positive/CD1a negative/CD4 single positive mature helper T cells in the cell populations obtained on day 35 of the culture schedule were compared among coating combinations (a), (c), (e), and (g). The results (mean ± standard error) are shown in Table 3 and Figure 5. The n values in Table 3 indicate the number of independent experiments. Statistical analysis was performed using a paired t-test. Coating combination (c) showed a statistically significantly higher percentage of CD3 and CD5 positive/CD1a negative/CD4 single positive mature helper T cells compared with coating combinations (a), (e), and (g) (Figure 5). It was shown that the combination of DLL4-Fc single coating in step (1) and the combination of DLL4-Fc and Retronectin double coating in step (2) were effective for producing CD4 single positive helper T cells.

**(Table 3)**

| **Process and culture schedule** | **Coating combination** | | |
|---|---|---|---|
| | (i) | (c) | (d) |
| **Process (1): Days 1 to 14** | D | D | D |
| **Process (2): Days 15 to 21** | D + R | D + R | D + R |
| **Process (3): Days 22 to 34** | - | R | D + R |
| **Percentage of existence of CD4SP IPSC-T cell (%)** | 2.39±0.75 (n=6) | 9.88±2.17 (n=10) | 1.92±0.97 (n=4) |

(Abbreviations used in the table)
D: DLL4-Fc
R: Retronectin
CD4SP iPSC-T cells: CD4 single-positive helper T cells derived from iPS cells

### Example 5

### [Effect of Wnt/β-catenin pathway activators on the production of CD4 single-positive helper T cells from iPS cells]

The effect of adding a Wnt/β-catenin pathway activator on the production of CD4 single-positive helper T cells was examined in steps (1), (2), and (3) shown in Example 1. The test was performed as described in Example 1, except that iPS cells transfected with a specific TCR were used. The coating conditions for DLL4-Fc and retronectin were as follows: coating combination (c) (see Tables 1-3). The GSK-3 inhibitor CHIR-99021 was used as the Wnt/β-catenin pathway activator, and was added at a concentration of 1 µM in steps (1), (2), and (3).

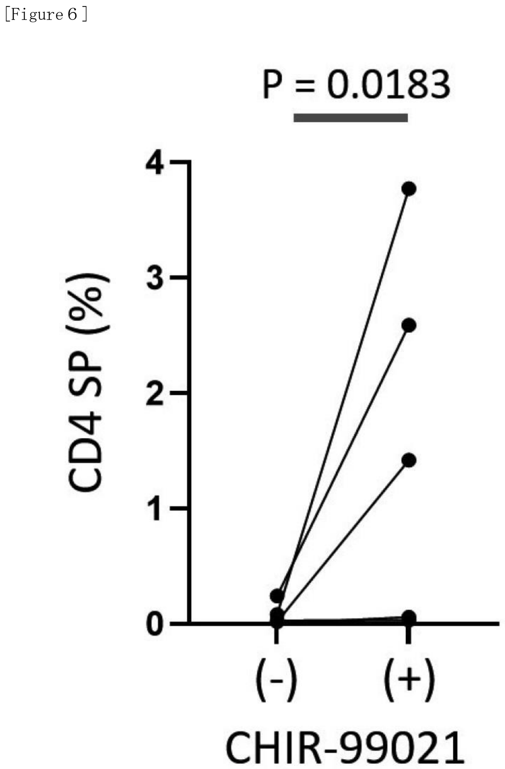

The percentages of CD3 and CD5 positive/CD1a negative/CD4 single positive mature helper T cells in the cell population obtained on day 35 of the culture schedule were compared in the presence and absence of CHIR-99021. Four independent tests were performed, and the results are shown in Figure 6. The percentage of CD4 single positive mature helper T cells was statistically significantly higher in the presence of CHIR-99021 than in the absence of CHIR-99021. Statistical analysis was performed using a paired t-test.

### Example 6

### [CD40L Expression upon TCR Stimulation of CD4 Single Positive Helper T Cells (CD4SP iPSC-T Cells) Produced from iPS Cells (1)]

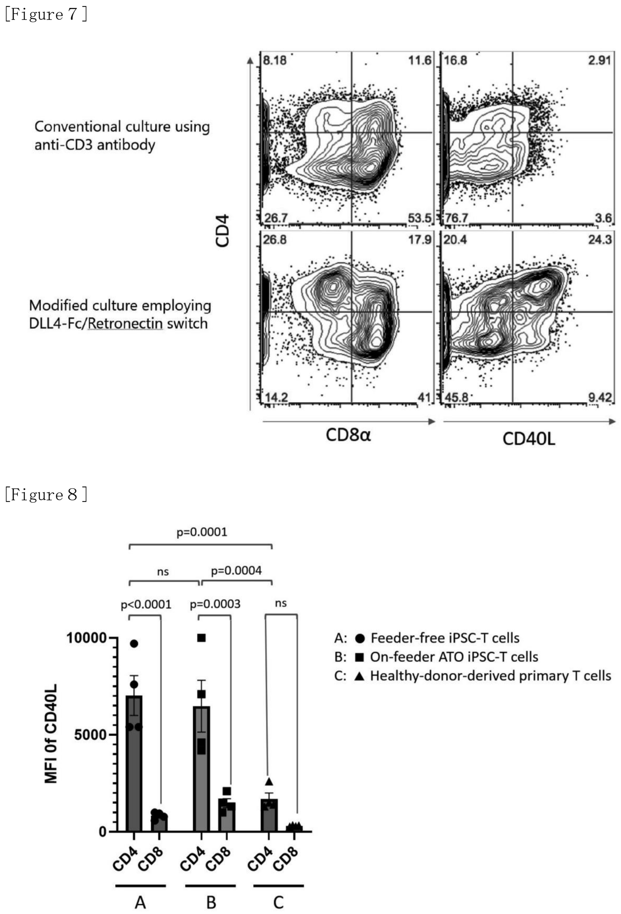

CD40L (CD40 ligand) is an effector molecule expressed primarily on activated CD4-positive T cells and is involved in the maturation of dendritic cells. CD40L expression in cell populations containing CD4SP iPSC-T cells matured by DLL4-Fc/retroNectin switching on cell culture plate coatings according to Example 1 was analyzed by flow cytometry and compared with conventional CD4SP iPSC-T cells matured from iPS cells using an anti-CD3 antibody (OKT3) as previously reported (WO 2018/135646). Flow cytometry analysis of the CD4SP iPSC-T cells matured by DLL4-Fc/retroNectin switching was performed 4 days after TCR stimulation with the anti-CD3 antibody coated on the cell culture plate in the presence of IL-7, IL-12, IL-15, IL-18, and IL-21. The analysis results are shown in Figure 7. In Figure 7, the upper panel shows the analytical results for conventional CD4SP iPSC-T cells matured with an anti-CD3 antibody, and the lower panel shows the analytical results for CD4SP iPSC-T cells matured by DLL4-Fc/retroNectin switching.

The results in Figure 7 indicate that CD4SP iPSC-T cells matured by DLL4-Fc/retronectin switching exhibited higher CD40L expression upon TCR stimulation than CD4SP iPSC-T cells matured with an anti-CD3 antibody. Specifically, CD4SP iPSC-T cells matured by DLL4-Fc/retronectin switching contained 24.3% highly CD40L-expressing cells, whereas CD4SP iPSC-T cells matured with an anti-CD3 antibody contained only 2.91% highly CD40L-expressing cells. This suggests that CD4SP iPSC-T cells matured by DLL4-Fc/retronectin switching also function better as helper T cells than conventional CD4SP iPSC-T cells matured with an anti-CD3 antibody.

### Example 7

### [CD40L Expression by TCR Stimulation of CD4 Single Positive Helper T Cells (CD4SP iPSC-T Cells) Produced from iPS Cells (2)]

CD4SP iPSC-T cells and CD8 SP iPSC-T cells were isolated from a cell population containing CD4SP iPSC-T cells and CD8 single positive killer T cells (CD8SP iPSC-T cells) that had been matured without feeder cells by DLL4-Fc/retronectin switching in the coating of cell culture plates, as described in Example 1. In addition, CD4 single-positive helper T cells (ATO-CD4SP iPSC-T cells) and CD8 single-positive killer T cells (ATO-CD8SP iPSC-T cells) were isolated from T cells produced from iPS cells by culturing them in an artificial thymic organ (ATO, see Non-Patent Document 6) on feeder cells. Furthermore, CD4 single-positive helper T cells and CD8 single-positive killer T cells were isolated from primary T cells derived from peripheral blood of healthy individuals. CD40L expression in these cells was analyzed by flow cytometry. Flow cytometry analysis was performed 24 hours after stimulation of each cell type with anti-CD3 antibody (1 µg/mL) immobilized on a cell culture plate in the presence of IL-7 (5 ng/mL), IL-12 (50 ng/mL), IL-15 (5 ng/mL), IL-18 (50 ng/mL), and IL-21 (20 ng/mL).

The CD40L expression of each cell type was measured as mean fluorescence intensity by flow cytometry analysis and is shown in Figure 8. The results in Figure 8 are expressed as mean ± standard error, and statistical analysis was performed using a paired t-test. CD4SP iPSC-T cells matured without the use of feeder cells were shown to express significantly more CD40L than feeder-free CD8SP iPSC-T cells, ATO-CD8SP iPSC-T cells, and primary T cells derived from healthy individuals (CD4 single-positive helper T cells and CD8 single-positive killer T cells). The CD40L expression level of feeder-free CD4SP iPSC-T cells was comparable to that of ATO-CD4SP iPSC-T cells. This suggests that CD4SP iPSC-T cells matured via DLL4-Fc/retronectin switching exhibit superior helper T cell function.

### Example 8

### [Thpok and PLZF Expression in CD4 Single Positive Helper T Cells (CD4SP iPSC-T Cells) and CD8 Single Positive Killer T Cells (CD8SP iPSC-T Cells) Produced from iPS Cells]

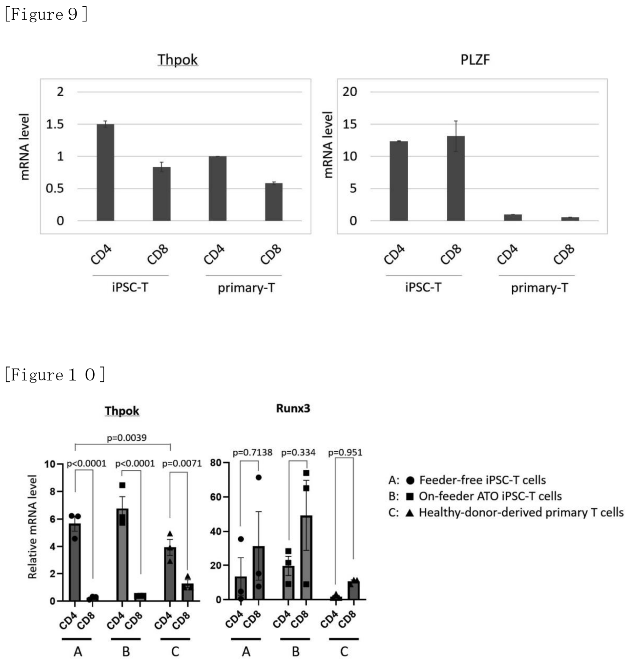

Thpok is a master transcription factor for helper T cell differentiation and is essential for helper T cell function. PLZF is a master transcription factor for the differentiation of innate immune T cells, such as natural killer T (NKT) cells. Mature CD4SP iPSC-T cells and CD8SP iPSC-T cells are obtained by culturing iPS cells using DLL4-Fc/retronectin switching in the coating of cell culture plates, as described in Example 1. The expression of these two transcription factors in these T cells was compared with that of primary T cells of the adaptive immune system obtained from peripheral blood. Expression of these transcription factors was measured by TaqMan qPCR (quantitative PCR). For Thpok measurement, the reagent set used was Assay ID: Hs00757087_g1 (Gene: Zbtb7b) (Thermo Fisher Scientific, Catalog No.: 4331182). For PLZF measurement, the reagent set used was Assay ID: Hs00957433_m1 (Gene: Zbtb16) (Thermo Fisher Scientific, Catalog No.: 4331182). Measurement results were normalized by the expression level of β-actin. The results are shown in Figure 9. Results are presented as mean ± standard deviation (n = 3).

Among primary T cells of the adaptive immune system obtained from peripheral blood, CD4 single-positive T cells had higher Thpok expression levels than CD8 single-positive T cells. Similarly, CD4SP iPSC-T cells had higher Thpok expression levels than CD8SP iPSC-T cells, suggesting that they exhibit a phenotype related to helper T cells. Meanwhile, both CD4SP iPSC-T cells and CD8SP iPSC-T cells express PLZF, which is significantly different from primary T cells of the adaptive immune system obtained from peripheral blood, which do not express PLZF at all. This suggests that CD4SP iPSC-T cells and CD8SP iPSC-T cells have a unique innate immune-like phenotype.

### Example 9

### [Thpok and Runx3 Expression in CD4 Single-Positive Helper T Cells (CD4SP iPSC-T Cells) and CD8 Single-Positive Killer T Cells (CD8SP iPSC-T Cells) Produced from iPSCs]

Thpok is the master transcription factor for helper T cell differentiation and is essential for helper T cell function. Runx3 is the master transcription factor for killer T cell differentiation. According to Example 1, CD4SP iPSC-T cells and CD8SP iPSC-T cells were isolated from a cell population containing CD4SP iPSC-T cells and CD8 single-positive killer T cells (CD8SP iPSC-T cells) that were matured without the use of feeder cells by DLL4-Fc/retronectin switching in the coating of cell culture plates. Also, CD4 single-positive helper T cells (ATO-CD4SP iPSC-T cells) and CD8 single-positive killer T cells (ATO-CD8SP iPSC-T cells) were isolated from T cells produced from iPS cells by culture in an artificial thymic organ (ATO; see Non-Patent Document 6) on feeder cells. Furthermore, CD4 single-positive helper T cells and CD8 single-positive killer T cells were isolated from primary T cells derived from peripheral blood of healthy donors. Total mRNA was isolated from these cells immediately after maturation, and Thpok and Runx3 expression was measured by TaqMan qPCR (quantitative PCR). Thpok was measured using the reagent set Assay ID: Hs00757087_g1 (Gene: Zbtb7b) (Thermo Fisher Scientific, Catalog No.: 4331182). Runx3 was measured using the reagent set Assay ID: Hs01091094_m1 (Gene: Runx3) (Thermo Fisher Scientific, Catalog No.: 4331182). Measurement results were normalized to β-actin expression levels. The results are shown in Figure 10. Results are presented as mean ± standard error (n = 3).

CD4SP iPSC-T cells matured without the use of feeder cells, like ATO-CD4SP iPSC-T cells and CD4 single-positive helper T cells derived from peripheral blood-derived primary T cells, had statistically significantly higher Thpok expression levels than their respective corresponding CD8 single-positive killer T cells, suggesting that they exhibit a helper T cell-related phenotype. Furthermore, the Thpok expression level of feeder-free CD4SP iPSC-T cells was significantly higher than that of peripheral blood-derived CD4 single-positive helper T cells, suggesting enhanced helper T cell function. On the other hand, no significant difference in Runx3 expression was observed between CD4 single-positive helper T cells and CD8 single-positive killer T cells.

### Example 10

### [Proliferation of CD4 single-positive helper T cells (CD4SP iPSC-T cells) and CD8 single-positive killer T cells (CD8SP iPSC-T cells) produced from iPS cells]

CD4SP iPSC-T cells and CD8SP iPSC-T cells were isolated from a cell population containing CD4SP iPSC-T cells and CD8 single-positive killer T cells (CD8SP iPSC-T cells) that were matured without the use of feeder cells by DLL4-Fc/retronectin switching in the coating of cell culture plates according to Example 1. In addition, CD4 single-positive helper T cells (ATO-CD4SP iPSC-T cells) and CD8 single-positive killer T cells (ATO-CD8SP iPSC-T cells) were isolated from T cells produced from iPS cells by culturing them in an artificial thymic organ (ATO, see Non-Patent Document 6) on feeder cells. Furthermore, CD4 single-positive helper T cells and CD8 single-positive killer T cells were isolated from primary T cells derived from peripheral blood of healthy individuals. These cells were stimulated with anti-CD3 antibody (1 µg/mL) immobilized on a cell culture plate in the presence of IL-7 (5 ng/mL), IL-12 (50 ng/mL), IL-15 (5 ng/mL), IL-18 (50 ng/mL), and IL-21 (20 ng/mL), and the cell count was measured 14 days later. The cell count was determined as viable cells by trypan blue staining.

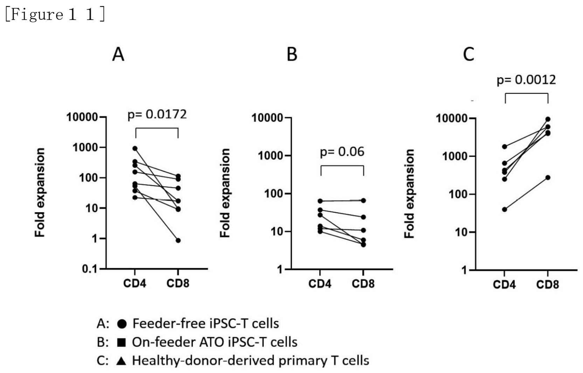

The proliferation rates of the above cells are shown in Figure 11. In Figure 11, dots representing pairs of independent experiments (CD4 and CD8) are connected by lines. The proliferation rate of feeder-free CD4SP iPSC-T cells was significantly higher than that of feeder-free CD8SP iPSC-T cells. Conversely, for peripheral blood-derived primary T cells, the proliferation rate of CD8 single-positive killer T cells was significantly higher than that of CD4 single-positive helper T cells. The proliferation rates of ATO-CD4SP iPSC-T cells and ATO-CD8SP iPSC-T cells were nearly identical.

### Example 11

### [Analysis of naive and adaptive immune markers expressed by CD4 single-positive helper T cells (CD4SP iPSC-T cells) and CD8 single-positive killer T cells (CD8SP iPSC-T cells) produced from iPS cells after feeder-free expansion]

Cell populations containing CD4SP iPSC-T cells and CD8SPC iPSC-T cells produced according to Example 1 were expanded without the use of feeder cells. Expansion was performed as follows: First, anti-CD3 antibody (OKT3) diluted to 1 µg/mL in PBS was added to each well of a 96-well flat-bottom cell culture plate. The plate was then incubated overnight at 4°C and washed once with PBS to prepare an anti-CD3 antibody-coated cell culture plate. Next, the cell population was suspended in αMEM containing 20% fetal bovine serum, PSG (penicillin-streptomycin-L-glutamine) solution (100x), ITS (insulin-transferrin-sodium selenite) supplement (100x), 2-phospho-L-ascorbic acid (50 µg/mL), human recombinant IL-7 (5 ng/mL), human recombinant IL-15 (5 ng/mL), human recombinant IL-21 (20 ng/mL), IL-12 (50 ng/mL), and IL-18 (50 ng/mL). The suspension was added to the anti-CD3 antibody-coated cell culture plates (2 x 10⁴ cells/200 µL/well) and cultured at 37°C in a 5% CO₂ environment for 16 hours. The cells were then transferred to cell culture plates that had not been coated with anti-CD3 antibody. After that, half of the culture medium was replaced with fresh medium every three days, and the cells were cultured for 14 days at 37°C in a 5% CO₂ environment. The culture medium used for the medium replacement was αMEM containing 20% fetal bovine serum, PSG solution (100x), ITS supplement (100x), 2-phospho-L-ascorbic acid (50 µg/mL), human recombinant IL-7 (5 ng/mL), and human recombinant IL-15 (5 ng/mL).

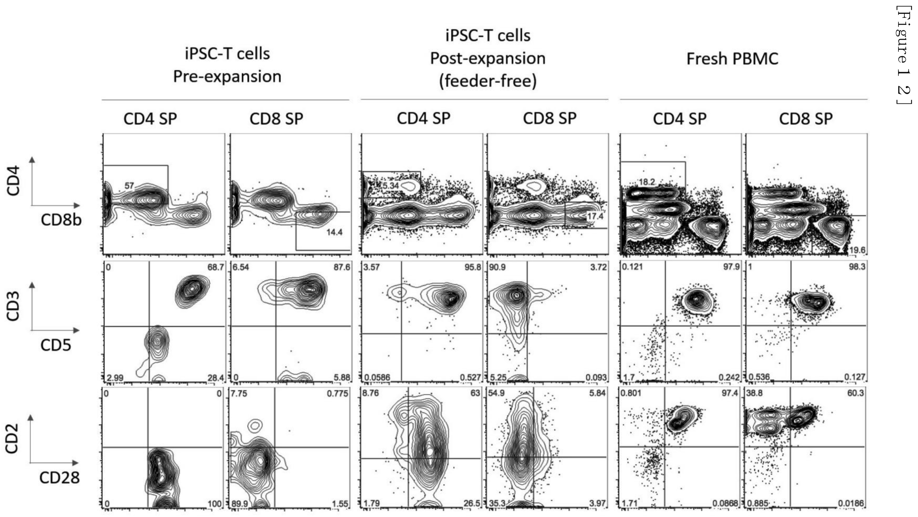

Cell surface markers were analyzed by flow cytometry for post-expansion cell populations and compared with pre-expansion cell populations. Fresh human peripheral blood mononuclear cells (PBMCs) were also analyzed by flow cytometry for cell surface markers. PBMCs were prepared using lymphocyte separation tubes (Greiner Bio-One) according to the manufacturer's instructions. The results are shown in Figure 12. CD2 and CD5 are established adaptive immune markers expressed by all T cells. Meanwhile, CD28 is a naive immune marker. After feeder-free expansion, Figure 12 shows that CD4SP iPSC-T cells expressed and maintained the expression of adaptive immune and naive markers compared to CD8SP iPSC-T cells, even after expansion. Furthermore, it was shown that the expression of CD5 and CD28 in CD4SP iPSC-T cells was equivalent to that in fresh PBMCs derived from healthy individuals, even after expansion. These adaptive immune and naive markers are highly expressed in young T cells with less exhausted traits, and are known to positively correlate with the efficacy of immune cell therapy.

### Example 12

### [Cytokine Production by CD4 Single Positive Helper T Cells (CD4SP iPSC-T Cells) and CD8 Single Positive Killer T Cells (CD8SP iPSC-T Cells) Produced from iPSCs]

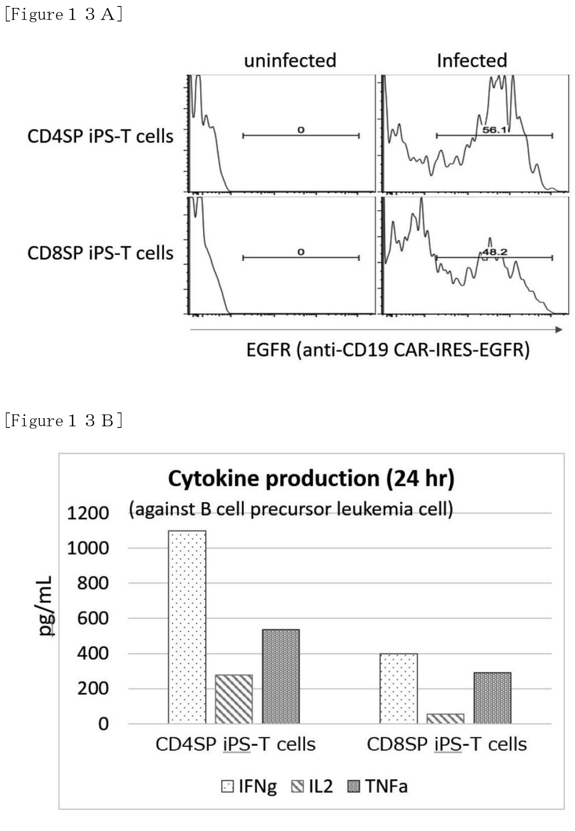

A cell population containing CD4SP iPSC-T cells and CD8SP iPSC-T cells produced according to Example 1 was retrovirally transduced with an anti-CD19 CAR (anti-CD19 CAR-IRES-EGFR) as previously reported (Ueda T, et al. Nat Biomed Eng. 2023;7:24-37). EGFR expression in the resulting cells was analyzed by flow cytometry. Figure 13A shows a histogram of EGFR expression, confirming EGFR expression in both CD4SP iPSC-T cells and CD8SP iPSC-T cells. CD4SP iPSC-T cells and CD8SP iPSC-T cells transfected with anti-CD19 CAR-IRES-EGFR were isolated by flow cytometry (FACSAria, Becton-Dixon). 1 × 104 cells each were co-cultured with the CD19-positive human pre-B cell leukemia cell line Nalm6 (5×10⁴ cells) at 37°C under 5% CO₂ for 24 hours. The co-culture was performed in αMEM (200 µL) containing 15% fetal bovine serum, PSG, and ITS in the presence of IL-7 (5 ng/mL) and IL-15 (5 ng/mL). After the culture, the cytokine levels (IFN-y, IL-2, and TNFα) in the culture supernatant were measured using the BD^{®} Cytometric Bead Array Human Th1/Th2/Th17 Cytokine Kit (BD Biosciences). The results are shown in Figure 13B. CD4SP iPSC-T cells were shown to produce higher levels of IFN-γ, IL-2, and TNFα compared to CD8SP iPSC-T cells. These cytokines not only exhibit direct antitumor activity but are also known to be able to modify the immunosuppressive tumor microenvironment. Therefore, it is suggested that CD4SP iPSC-T cells produced according to Example 1 may be useful for cancer immunotherapy.

### Example 13

### [Cancer Cell-Cytotoxic Effect of CD4 Single-Positive Helper T Cells (CD4SP iPSC-T Cells) and CD8 Single-Positive Killer T Cells (CD8SP iPSC-T Cells) Produced from iPS Cells]

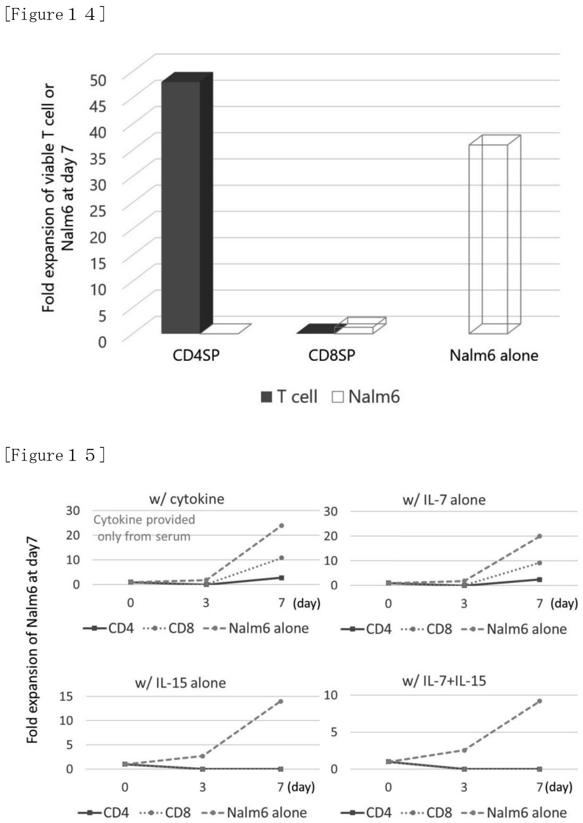

CD4SP iPSC-T cells and CD8SP iPSC-T cells were isolated by flow cytometry (FACSAria, Becton-Dixon) from a cell population containing CD4SP iPSC-T cells and CD8SP iPSC-T cells produced according to Example 1. These cells were used as effector cells (E) and as target cells (T) to examine their killing activity against the pre-B cell leukemia cell line Nalm6. CD4SP iPSC-T cells or CD8SP iPSC-T cells were co-cultured with Nalm6 cells in α-MEM containing 15% fetal bovine serum, PSG, and ITS in the presence of IL-7 (5 ng/mL) and IL-15 (5 ng/mL) at 37°C and 5% CO2. Co-culture was initiated at an E/T ratio of 0.25. On day 3, Nalm6 cells were added and continued at an E/T ratio of 0.125. On day 7, cytotoxicity against Nalm6 cells and proliferation of CD4SP iPSC-T cells or CD8SP iPSC-T cells were measured by FACS. The results are shown in Figure 14. On day 7 of co-culture, CD4SP iPSC-T cells were superior to CD8SP iPSC-T cells in proliferation and killing activity against Nalm6 cells.

### Example 14

### [Effect of IL-7 and IL-15 on the cytotoxic activity of CD4 single-positive helper T cells (CD4SP iPSC-T cells) and CD8 single-positive killer T cells (CD8SP iPSC-T cells) produced from iPS cells]

CD4SP iPSC-T cells and CD8SP iPSC-T cells were isolated from the cell population containing CD4SP iPSC-T cells and CD8SP iPSC-T cells prepared according to Example 1. These cells were used as effector cells (E) and the effects of IL-7 and IL-15 on the killing of the pre-B cell leukemia cell line Nalm6 as target cells (T) were examined. CD4SP iPSC-T cells or CD8SP iPSC-T cells (4 × 10³cells) were co-cultured with Nalm6 cells (2 × 10⁴ cells) using α-MEM containing 15% fetal bovine serum, PSG and ITS, in the presence or absence of IL-7 (5 ng/mL) and IL-15 (5 ng/mL), at 37°C in 5% CO₂ (E/T = 0.2). On day 3 of co-culture, additional Nalm6 cells (2 × 10⁴ cells) were added and co-culture was continued. The number of live Nalm6 cells was measured by FACS at the start of co-culture, on day 3, and on day 7. The results are shown in Fig. 15. In the presence of IL-15 alone and in the presence of both IL-7 and IL-15, the killing activity of CD4SP iPSC-T cells and CD8SP iPSC-T cells against Nalm6 cells was almost equivalent. In contrast, under serum only conditions (no IL-7 or IL-15 added) and in the presence of IL-7 alone, the cytotoxic activity of CD4SP iPSC-T cells was stronger than that of CD8SP iPSC-T cells. In other words, the cytotoxic activity of CD4SP iPSC-T cells is less dependent on the homeostatic cytokines IL-7 and IL-15 than that of CD8SP iPSC-T cells. This is a necessary property for T cells to retain their activity in tumor microenvironments where supply of homeostatic cytokines is insufficient.

### Example 15

### [Cytotoxic activity of CD4 single-positive helper T cells produced from iPS cells (CD4SP iPSC-T cells), CD8 single-positive killer T cells produced from iPS cells (CD8SP iPSC-T cells), T cells produced from iPS cells by artificial thymic organ (ATO) culture (ATO-iPSC-T cells; see non-patent literature 6), and primary T cells derived from healthy donors, against cancer cells]

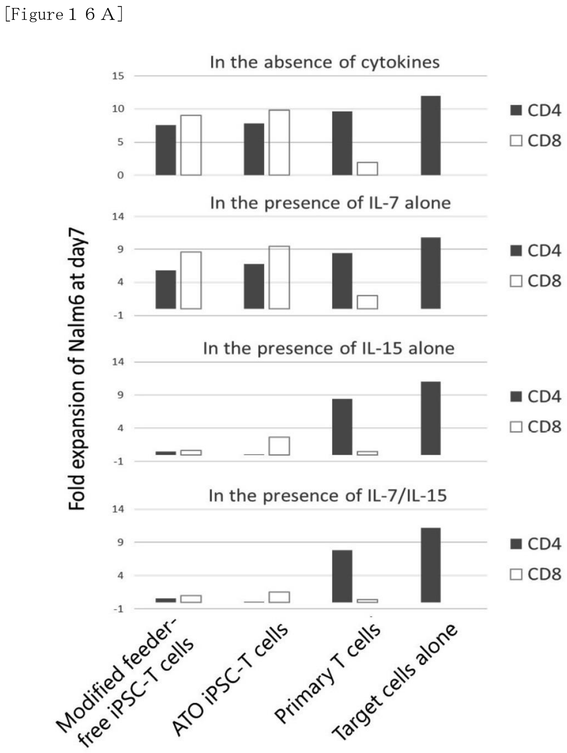

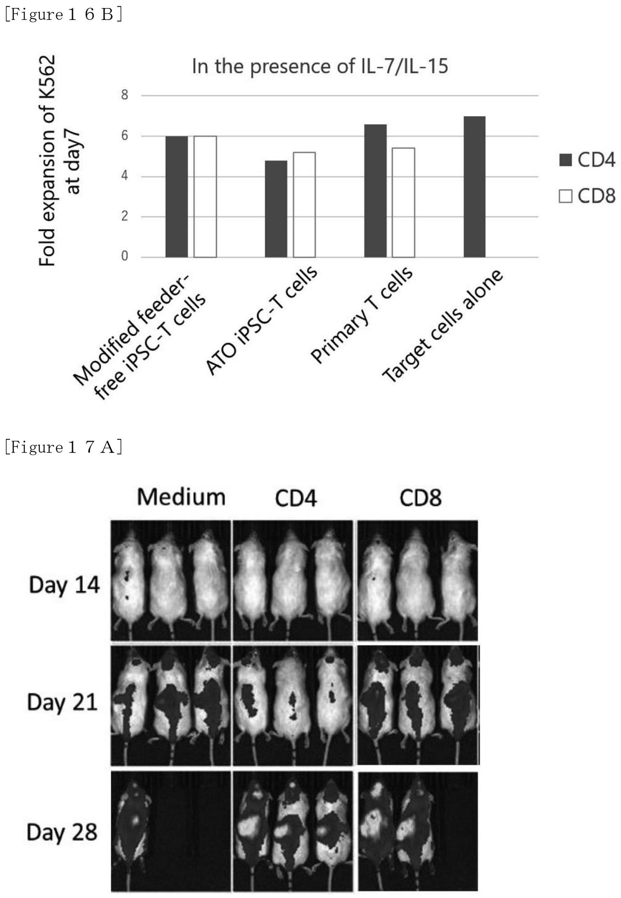

The CD4SP iPSC-T cells and CD8SP iPSC-T cells were produced according to Example 1 and expanded in culture without using feeder cells (modified feeder-free iPSC-T cells). Expansion was carried out according to Example 5. ATO-iPSC-T cells and primary T cells from healthy donors were expanded in the same manner. After expansion, these T cells were transduced with an anti-CD19 CAR (anti-CD19 CAR-IRES-EGFR) using a retrovirus according to a previous report (Ueda T, et al. Nat Biomed Eng. 2023; 7:24-37). CD4SP and CD8SP T cells expressing the anti-CD19 CAR (EGFR-positive) were isolated by flow cytometry (FACSAria, Becton Dickinson). These T cells (4 × 10³) were used as effector cells (E) and were co-cultured with target cells (T)-either the CD19-positive human pre-B cell leukemia line Nalm6 or the CD19-negative human leukemia line K562 (2 × 10⁴)-at 37°C in 5% CO2. On day 3 of co-culture, additional target cells (2 × 10⁴) were added and co-culture was continued; on day 7 the growth of the cancer cells (target cells) was measured by FACS. Co-cultures were performed in α-MEM containing 15% fetal bovine serum, PSG and ITS under conditions with no cytokines, with IL-7 (5 ng/mL), with IL-15 (5 ng/mL), and with both IL-7 and IL-15. After co-culture, viable Nalm6 and K562 cell numbers were measured by FACS. Results with Nalm6 as targets are shown in Fig. 16A, and results with K562 as targets are shown in Fig. 16B.

The modified feeder-free iPSC-T cells exhibited cytotoxic activity against CD19-positive Nalm6 cells comparable to that of ATO-iPSC-T cells. CD4SP T cells derived from both the modified feeder-free iPSC-T cells and the ATO-iPSC-T cells showed superior target-specific cytotoxic activity compared with CD8SP T cells. In contrast, among primary T cells, in vitro cytotoxic activity was observed only in CD8SP T cells. Unlike the CD8SP iPSC-T cells produced in previous reports (Maeda T, et al. Cancer Res. 2016; 76:6839-6850; Kawai Y, et al. Mol Ther. 2021; 29:3027-3041), the modified feeder-free iPSC-T cells, ATO-iPSC-T cells, and primary T cells did not exhibit NK activity against NK-sensitive K562 cells. This suggests that, unlike the T cells produced in the cited reports, these cells have a lower risk of graft-versus-host disease caused by unintended NK activity.

### Example 16

### [Effect of CD4 single-positive helper T cells produced from iPS cells (CD4SP iPSC-T cells) and CD8 single-positive killer T cells (CD8SP iPSC-T cells) produced from iPS cells in mice transplanted with the human pre-B cell leukemia line Nalm6: impact on in vivo Nalm6 dynamics and mouse survival]

NOD-SCID IL2Rγc null (NSG) mice were intravenously inoculated with luciferase-expressing human pre-B cell leukemia Nalm6 cells (2 × 10⁵ cells per mouse) (day 0). On day 2, CD4SP iPSC-T cells or CD8SP iPSC-T cells (5 × 10⁶ cells per mouse), prepared according to Example 1, expanded in feeder-free culture, and carrying an anti-CD19 CAR, were administered by intravenous injection. As a control, culture medium without the above T cells was administered intravenously. Then on days 14, 21 and 28, luciferase emission was monitored by in vivo imaging. The results are shown in Fig. 17A. In the control group, luciferase signal increased over time, indicating that the transplanted Nalm6 cells engrafted and proliferated. In contrast, the CD4SP iPSC-T cell-treated group showed lower luciferase levels than the control group, demonstrating suppression of Nalm6 growth. The growth-suppressive effect of the CD4SP iPSC-T cell treatment was stronger than that observed in the CD8SP iPSC-T cell-treated group.

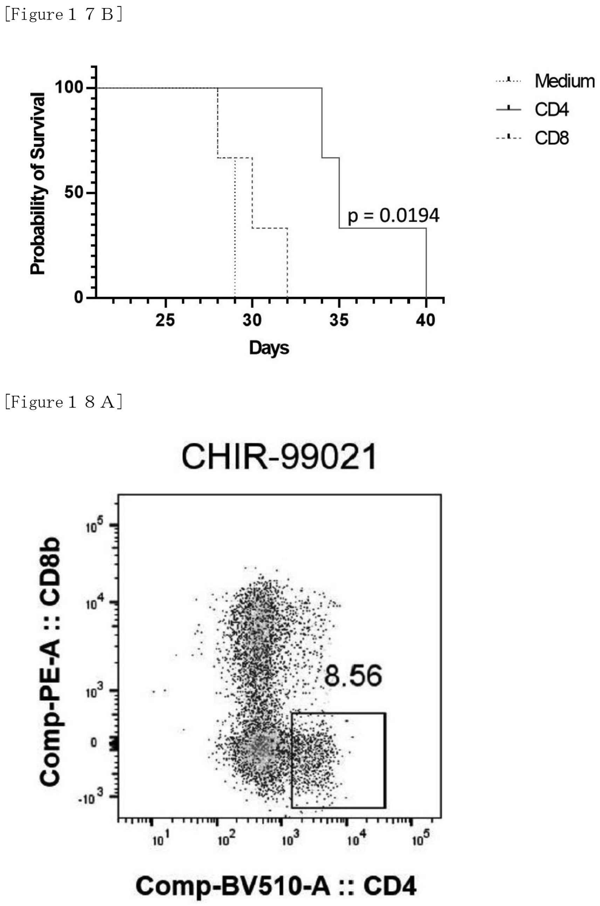

Figure 17B shows the Kaplan-Meier survival curve. CD4SP iPSC-T cells demonstrated significantly improved survival rates compared to CD8SP iPSC-T cells, demonstrating their superior in vivo tumor suppression effects. Statistical significance was analyzed using the log-rank test with the Maltel-Cox method (*P=0.0194).

### Example 17

### [Production of CD4 Single Positive Helper T Cells from iPS Cells (Method 2)]

iPS cells were produced from CD3-positive T cells isolated from human peripheral blood using the method described in Non-Patent Document 3. The T cells were isolated using a whole T cell isolation kit (Pan T Cell Isolation Kit, Miltenyi Biotec, Catalog No.: 130-096-535).

### (Day 0)

150 µL of DLL4-Fc solution (Sino Bio, Catalog No. 10171-H02H) diluted to 5 µg/mL in PBS (phosphate-buffered saline) was added to each well of a 48-well cell culture plate (Corning, Catalog No. 3548) and allowed to stand overnight at 4°C to coat the cell culture plate with human DLL4-Fc. DLL4-Fc is a recombinant protein in which the Fc domain of human IgG1 is attached to the C-terminus of the extracellular domain (residues 1-524) of human DLL4 (Delta-Like Protein 4). It was used as a T cell differentiation inducing factor.

### Days 1-21: Step (1)

### (Day 1)

Embryoid bodies formed by culturing iPS cells in suspension for 14 days were collected and dissociated into single cells by aspirating and expelling six times with a syringe fitted with a 21-gauge needle, then cell debris was removed using a cell strainer. The resulting cells were suspended in T cell differentiation medium. DLL4-Fc-coated culture plates were washed once with 1 mL/well PBS, then 500 µL/well of T cell differentiation medium was added. To this, 500 µL/well of the cell suspension containing 2 × 10³ cells was added. The cells on the culture plates were maintained at 37°C in 5% CO₂, with half of the medium replaced with fresh T cell differentiation medium every 2-3 days.

As the T cell differentiation medium, α-MEM (Thermo Fisher Scientific) was used containing: 15% fetal bovine serum (Corning); ITS (insulin-transferrin-selenium) supplement (100X); 2-mercaptoethanol (final 55 µM, Thermo Fisher Scientific); the GSK-3 inhibitor CHIR-99021 (1 µM, Wako); 2-phospho-L-ascorbic acid (final 50 µg/mL); GlutaMAX (100×); human recombinant SCF (final 50 ng/mL); human recombinant TPO (final 50 ng/mL); human recombinant IL-7 (final 50 ng/mL); human recombinant Flt3L (final 50 ng/mL); human recombinant SDF-1α (final 10 nM, PeproTech); and the p38 inhibitor SB203580 (final 15 µM, Tocris Bioscience).

### (Day 21)

The obtained cells were analyzed by flow cytometry, and it was confirmed that the cells differentiated from iPS cells were CD4/CD8 double positive.

### Days 22-23: Step (2)

### (Day 22)

PBS containing 5 µg/mL RetroNectin (RetroNectin, Registered Trademark, Takara Bio) was added to each well of a new 48-well cell culture plate and left at 37°C for 2 hours to coat the cell culture plate with RetroNectin. RetroNectin was used as a cell adhesion factor.

After washing the RetroNectin-coated cell culture plates once with 1 mL/well PBS, the CD4/CD8 double-positive cells were added and stimulated with 1 µg/mL of an anti-CD3 agonist antibody (OKT3) for 2 days. The culture medium used was αMEM containing 15% fetal bovine serum, ITS (insulin-transferrin-sodium selenite) supplement (100×), GlutaMAX (100×), 2-phospho-L-ascorbic acid (50 µg/mL), PSG (penicillin-streptomycin-L-glutamine) solution (100×, Sigma-Aldrich), the GSK-3 inhibitor CHIR-99021 (1 µM, Wako), IL-7 (50 ng/mL), SDF-1α (10 nM) and the p38 inhibitor SB203580 (10 mM).

### Day 24 onwards (Days 27-34): Step (3)

### (Day 24)

The cells obtained in step (2) were collected, washed, and then seeded onto freshly prepared cell culture plates coated with Retronectin. The cells were cultured for 4 to 11 days at 37°C in a 5% CO₂ environment using a culture medium with the same composition as that used in step (2) (but without anti-CD3 antibody).

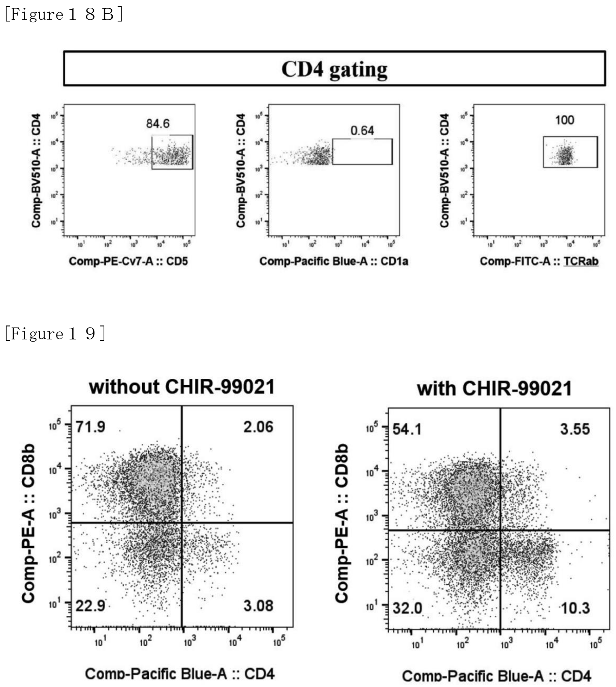

Flow cytometric analysis of the cells obtained in step (3) confirmed the presence of CD4 single-positive helper T cells (Figure 18A). Further flow cytometric analysis of the CD4 single-positive helper T cell subset showed high expression of CD5 and TCR α/β chains, but almost no expression of CD1a (Figure 18B).

### [Example 18]

### [Effect of the GSK-3 inhibitor CHIR-99021 on production of CD4 single-positive helper T cells and CD8 single-positive killer T cells]

The GSK-3 inhibitor CHIR-99021 functions as a Wnt pathway activator. In steps (1)-(3) of Example 17, production of CD4 single-positive helper T cells from iPS cell-derived hematopoietic stem cells was examined in the presence or absence of CHIR-99021. The cells obtained were analyzed by flow cytometry, and the results are shown in Fig. 19. The proportion of CD4 single-positive helper T cells was 3.08% in the absence of CHIR-99021 and 10.3% in the presence of CHIR-99021, demonstrating that addition of CHIR-99021 promoted production of CD4 single-positive helper T cells.

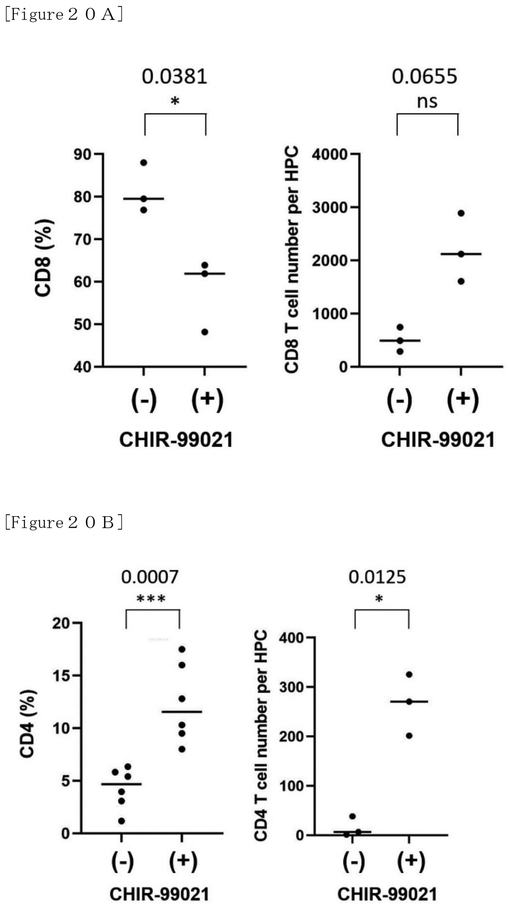

Next, the abundance and cell yield of CD4 single-positive helper T cells and CD8 single-positive killer T cells were examined in the presence or absence of CHIR-99021 in steps (1) to (3) of Example 17. The results are shown in Figures 20A and 20B. CHIR-99021 significantly increased the abundance of CD4 single-positive helper T cells (Figure 20B), but significantly decreased the abundance of CD8 single-positive killer T cells (Figure 20A). Meanwhile, cell yield increased for both CD4 single-positive helper T cells and CD8 single-positive killer T cells. Statistical analysis was performed using a paired t-test.

### Example 19

### [CD4 and CD8 Expression After Expansion of CD4 and CD8 Single-Positive T Cells Obtained in the Presence of CHIR-99021]

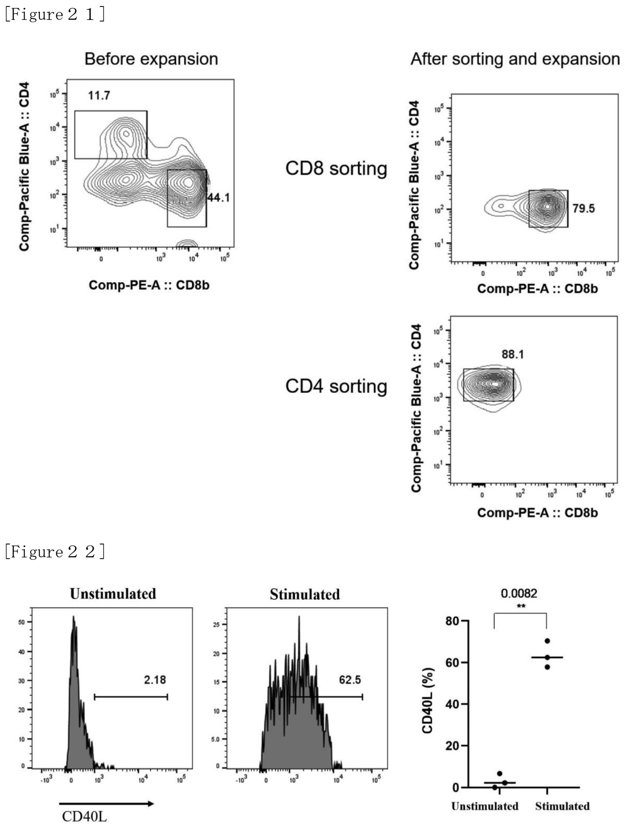

Following Example 17, CD4 and CD8 expression in mature T cells produced from iPS cell-derived hematopoietic stem cells in the presence of CHIR-99021 was analyzed by flow cytometry. The results are shown in Figure 21 (Before expansion). These results confirmed the production of CD4 single-positive T cells and CD8 single-positive T cells. These CD4 and CD8 single-positive T cells were sorted by flow cytometry and expanded for 14 days using anti-CD3 and anti-CD28 antibody-conjugated beads (Dynabeads^{®}), after which CD4 and CD8 expression was analyzed by flow cytometry. The results are shown in Figure 21 (After sorting and expansion). These results demonstrated that the resulting CD4 single-positive T cells and CD8 single-positive T cells could both be expanded and maintained the expression of their co-receptors.

### Example 20

### [CD40L Expression by TCR Stimulation of CD4 Single Positive Helper T Cells (CD4SP iPSC-T Cells) Produced from iPS Cells in the Presence of CHIR-99021]

A cell population containing CD4SP iPSC-T cells was produced from iPS cells according to Example 17. CD40L expression in this cell population was analyzed by flow cytometry before and after TCR stimulation with an anti-CD3 agonist antibody coated on a cell culture plate in the presence of IL-7, IL-12, IL-15, IL-18, and IL-21. The analysis results are shown in Figure 22. CD40L-expressing cells increased from 2.18% to 62.5% upon TCR stimulation. Comparison of the percentage of CD40L-expressing cells before and after TCR stimulation in three independent experiments showed that TCR stimulation significantly increased the number of CD40L-expressing cells. This suggests that the CD4SP iPSC-T cells produced according to Example 17 fully function as helper T cells upon TCR stimulation. Statistical analysis was performed using a paired t-test.

### Example 21

### [Cytokine Production by CD4 Single Positive Helper T Cells (CD4SP iPSC-T Cells) and CD8 Single Positive Killer T Cells (CD8SP iPSC-T Cells) Produced from iPS Cells in the Presence of CHIR-99021]

CD4SP iPSC-T cells and CD8SP iPSC-T cells produced according to Example 17 were treated with phorbol 12-myristate 13-acetate (PMA) and ionomycin in the presence of monensin for 4 hours, then stained with anti-IFN-γ and anti-IL-2 antibodies to measure cytokine production. The results are shown in Figure 23. CD4SP iPSC-T cells were shown to produce higher levels of IFN-γ and IL-2 compared to CD8SP iPSC-T cells. These cytokines not only exhibit direct antitumor activity, but are also known to be able to modify the immunosuppressive tumor microenvironment. Therefore, it is suggested that CD4SP iPSC-T cells produced according to Example 17 may be useful for cancer immunotherapy.

### Example 22

### [Anti-CD19 CAR gene transfer into CD4 single-positive helper T cells (CD4SP iPSC-T cells) and CD8 single-positive killer T cells (CD8SP iPSC-T cells) produced from iPS cells in the presence of CHIR-99021]

CD4 single-positive helper T cells and CD8 single-positive killer T cells prepared according to Example 17 were retrovirally transduced with anti-CD19 CAR (anti-CD19 CAR-4-1 BB-CD3zeta) as previously reported (Ueda T, et al. Nat Biomed Eng. 2023; 7:24-37). Expression of anti-CD19 CAR in the resulting cells was analyzed by flow cytometry. The results are shown in Figure 24. It was confirmed that anti-CD19 CAR was expressed to similar levels in both CD4SP iPSC-T cells and CD8SP iPSC-T cells.

### Example 23

### [Cancer Cell Killing Effect of CD4 Single Positive Helper T Cells (CD4SP iPSC-T Cells) and CD8 Single Positive Killer T Cells (CD8SP iPSC-T Cells) Produced from iPS Cells in the Presence of CHIR-99021]

CD4SP iPSC-T cells and CD8SP iPSC-T cells produced according to Example 17 were transfected with anti-CD19 CAR (anti-CD19 CAR-4-1 BB-CD3zeta) and used as effector cells (E). The CD19-positive human pre-B cell leukemia cell line Nalm6 was used as target cells (T) to examine the killing activity of effector cells against target cells. Effector cells (E):target cells (T) were co-cultured at varying ratios of 1:4, 1:2, 1:1, 2:1, and 4:1. After one day, the remaining target cells (Nalm6) were counted by flow cytometry. The results are shown in Figure 25. The killing activity of the cells was observed in both CD4SP iPSC-T cells and CD8SP iPSC-T cells.

## Claims

1. A method for producing a cell population containing CD4 single positive, CD8 negative helper T cells, comprising:
(1) culturing hematopoietic stem cells differentiated from pluripotent stem cells in the presence of a T cell differentiation inducing factor;
(2) culturing the cells obtained in step (1) in the presence of a T cell differentiation inducing factor and a cell adhesion factor; and
(3) culturing the cells obtained in step (2) in the presence of the cell adhesion factor but in the absence of the T cell differentiation inducing factor.

2. The method according to claim 1, wherein the cell culture in steps (1), (2), and (3) is performed in the presence of a Wnt/β-catenin pathway activator.

3. The method according to claim 1 or 2, further comprising a step of confirming that the cells obtained in step (1) contain CD3, CD1a, and CD5 positive T precursor cells.

4. The method according to claim 1 or 2, further comprising the step of confirming that the cells obtained in step (2) contain CD3 positive and CD4/CD8 double positive T cells.

5. The method according to claim 4, wherein the CD3 positive and CD4/CD8 double positive T cells are further CD5 and CD1a positive.

6. The method according to claim 1 or 2, wherein the cells obtained in step (3) are mature helper T cells that are CD1a and CD8 negative, and CD5 positive and CD4 single positive, or a mixture of the mature helper T cells and mature killer T cells that are CD5 and CD8 positive, and CD1a and CD4 negative.

7. The method according to claim 1 or 2, further comprising the step of confirming that the cells obtained in step (3) contain T cells that highly express CD28.

8. The method according to claim 7, wherein the CD28 expression level of the T cells that highly express CD28 is equivalent to the CD28 expression level of T cells present in human peripheral blood.

9. A method for producing a cell population comprising CD4 single positive and CD8 negative helper T cells, comprising:
(1) culturing hematopoietic stem cells differentiated from pluripotent stem cells in the presence of T cell differentiation-inducing factors and a Wnt/β-catenin pathway activator;
(2) culturing the cells obtained in step (1) in the presence of a cell adhesion factor, a Wnt pathway activator and an anti-CD3 antibody, and in the absence of T cell differentiation-inducing factors; and
(3) culturing the cells obtained in step (2) in the presence of a cell adhesion factor and a Wnt pathway activator, and in the absence of T cell differentiation-inducing factors and the anti-CD3 antibody.

10. The method according to claim 9, wherein the cells obtained in step (3) are a mixture of mature helper T cells that are CD1a and CD8 negative, and CD5 positive and CD4 single positive, and mature killer T cells that are CD5 and CD8 positive, and CD1a and CD4 negative.

11. The method according to claim 1 or 9, wherein the T cell differentiation inducing factor is a Notch ligand.

12. The method according to claim 11, wherein the Notch ligand is DLL4 or DLL4-Fc.

13. The method according to claim 1 or 9, wherein the cell adhesion factor is fibronectin or a fragment thereof, or RetroNectin (registered trademark).

14. The method according to claim 2 or 9, wherein the Wnt pathway activator is a GSK-3 inhibitor.

15. The method according to claim 14, wherein the GSK-3 inhibitor is CHIR-99021.

16. The method according to claim 1 or 9, wherein the T cell differentiation-inducing factor and the cell adhesion factor are present by being coated on a cell culture substrate.

17. The method according to claim 16, wherein the cell culture substrate is cell culture beads or a cell culture plate.

18. The method according to claim 1 or 2, wherein the cell culture in steps (1) and (2) is performed in the presence of IL-7 and FLT3L.

19. The method according to claim 1 or 2, wherein the cell culture in steps (1) and (2) is performed in the presence of IL-7, FLT3L, 2-phospho-L-ascorbic acid, and SDF-1α and/or a p38 inhibitor.

20. The method according to claim 1 or 2, wherein the cell culture in steps (1) and (2) is performed in the presence of IL-7, FLT3L, SDF-1α, and a p38 inhibitor.

21. The method according to claim 1 or 2, wherein the cell culture in steps (1) and (2) is performed in the presence of IL-7, FLT3L, 2-phospho-L-ascorbic acid, SDF-1α, a p38 inhibitor, and SCF.

22. The method according to claim 1 or 2, wherein the cell culture in step (3) is performed in the presence of IL-7.

23. The method according to claim 1 or 2, wherein the cell culture in step (3) is performed in the presence of IL-7, 2-phospho-L-ascorbic acid, and an SDF-1α and/or p38 inhibitor.

24. The method according to claim 1 or 2, wherein the cell culture in step (3) is performed in the presence of IL-7, SDF-1α, and a p38 inhibitor.

25. The method according to claim 1 or 2, wherein the cell culture in step (3) is performed in the presence of IL-7, 2-phospho-L-ascorbic acid, SDF-1α, a p38 inhibitor, and SCF.

26. The method according to claim 1 or 2, wherein the cell culture in steps (1) and (2) is performed while maintaining the cell density at subconfluence.

27. The method according to claim 9, wherein the cell culture in step (1) is performed in the presence of IL-7, FLT3L, 2-phospho-L-ascorbic acid, SDF-1α, a p38 inhibitor, SCF, and TPO.

28. The method according to claim 9, wherein the cell culture in steps (2) and (3) is performed in the presence of IL-7, 2-phospho-L-ascorbic acid, SDF-1α, and a p38 inhibitor.

29. The method according to claim 1 or 9, wherein the pluripotent stem cells are human iPS cells.

30. The method according to claim 29, wherein the human iPS cells are human peripheral blood mononuclear cells from which B cells and T cells have been depleted, or iPS cells obtained by reprogramming T cells.

31. A pharmaceutical agent comprising a cell population produced by the method according to claim 1 or 9.

32. The pharmaceutical according to claim 31, for use in the prevention or treatment of cancer.

33. An agent for killing cells expressing a tumor-associated antigen, comprising a cell population produced by the method according to claim 1 or 9.

34. A method for the prevention or treatment of cancer in a mammal, comprising administering to the mammal an effective amount of a cell population produced by the method according to claim 1 or 9.

35. A method for preventing or treating cancer in a mammal, comprising administering to the mammal an effective amount of the pharmaceutical agent of claim 31.

36. A method for preventing or treating cancer in a mammal, comprising administering to the mammal an effective amount of the agent according to claim 33.

37. A preventive or therapeutic agent for cancer in a mammal, comprising a cell population produced by the method according to claim 1 or 9.

38. A cell produced by the method according to claim 1 or 9 for use in the prevention or treatment of cancer.

39. A cell produced by the method according to claim 1 or 9 for use in the production of a cancer preventive or therapeutic agent.
